(19) Europäisches Patentamt
European Patent Office
Office européen des brevets

(11) **EP 3 010 933 B1**

(12) **FASCICULE DE BREVET EUROPEEN**

(45) Date de publication et mention de la délivrance du brevet:
**15.05.2024 Bulletin 2024/20**

(21) Numéro de dépôt: **14737174.4**

(22) Date de dépôt: **20.06.2014**

(51) Classification Internationale des Brevets (IPC):
**C07K 16/18** (2006.01)    **G01N 33/68** (2006.01)

(52) Classification Coopérative des Brevets (CPC):
**C07K 16/18; G01N 33/6875; G07C 1/30;**
C07K 2317/569

(86) Numéro de dépôt international:
**PCT/EP2014/062996**

(87) Numéro de publication internationale:
**WO 2014/202745 (24.12.2014 Gazette 2014/52)**

(54) **ANTICORPS MONOCATÉNAIRE À CHAÎNE LOURDE DE CAMÉLIDÉ DIRIGÉ CONTRE LA CHROMATINE ET UTILISATIONS**

ANTIKÖRPER MIT SCHWEREN EINZELKETTEN GEGEN CHROMATIN UND VERWENDUNGEN DAVON

CAMELID SINGLE HEAVY-CHAIN ANTIBODY DIRECTED AGAINST CHROMATIN AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.06.2013 FR 1355941**

(43) Date de publication de la demande:
**27.04.2016 Bulletin 2016/17**

(73) Titulaires:
• **Institut national de recherche pour l'agriculture, l'alimentation et l'environnement**
**75007 Paris (FR)**
• **Université Paul Sabatier Toulouse III**
**31400 Toulouse (FR)**

(72) Inventeurs:
• **MIREY, Gladys**
**31770 Colomiers (FR)**
• **JULLIEN, Denis**
**31520 Ramonville Saint Agne (FR)**
• **VIGNARD, Julien**
**31820 Pibrac (FR)**
• **SALLES, Bernard**
**31500 Toulouse (FR)**

(74) Mandataire: **Ipside**
**6, Impasse Michel Labrousse**
**31100 Toulouse (FR)**

(56) Documents cités:
**EP-A1- 1 433 793          EP-A1- 2 332 987**
**WO-A1-85/05689          WO-A1-99/35292**
**WO-A1-2006/079372    WO-A1-2008/156613**
**WO-A1-2009/016100    WO-A1-2009/061918**
**WO-A1-2011/131772    WO-A1-2012/156219**
**WO-A2-02/48193          WO-A2-2006/105142**
**WO-A2-2007/068313    WO-A2-2009/147201**
**WO-A2-2010/033667    WO-A2-2011/034580**
**WO-A2-2013/030578    US-A1- 2008 241 845**
**US-A1- 2014 017 673**

• **PICHLER GARWIN ET AL: "Fluorescent protein specific Nanotraps to study protein-protein interactions and histone-tail peptide binding", METHODS IN MOLECULAR BIOLOGY, HUMANA PRESS, INC, US, vol. 911, 1 janvier 2012 (2012-01-01), pages 475-483, XP009176629, ISSN: 1940-6029**

- ARBABI GHAHROUDI M ET AL: "Selection and identification of single domain antibody fragments from camel heavy-chain antibodies", FEBS LETTERS, ELSEVIER, AMSTERDAM, NL, vol. 414, no. 3, 15 septembre 1997 (1997-09-15), pages 521-526, XP004261105, ISSN: 0014-5793, DOI: 10.1016/S0014-5793(97)01062-4 cité dans la demande
- CAZALES MARTINE ET AL: "A new mitotic-cell specific monoclonal antibody", CELL CYCLE, LANDES BIOSCIENCE, US, vol. 7, no. 2, 1 janvier 2008 (2008-01-01), pages 267-268, XP008089675, ISSN: 1538-4101
- Christine Didier ET AL: "Evaluation of checkpoint kinase targeting therapy in acute myeloid leukemia with complex karyotype", Cancer Biology & Therapy, 1 mars 2012 (2012-03-01), pages 307-313, XP055139862, DOI: 10.4161/cbt.13.5.19074 Extrait de l'Internet: URL:http://www.pubmedcentral.nih.gov/artic lerender.fcgi?artid=3367716&tool=pmcentrez &rendertype=abstract [extrait le 2014-09-12]
- JULIEN VIGNARD ET AL: "Ionizing-radiation induced DNA double-strand breaks: A direct and indirect lighting up", RADIOTHERAPY AND ONCOLOGY, vol. 108, no. 3, 1 septembre 2013 (2013-09-01), pages 362-369, XP055139781, ISSN: 0167-8140, DOI: 10.1016/j.radonc.2013.06.013

Remarques:
Le document complet y compris le(s) table(s) de référence et le(s) listage(s) de séquence sont téléchargeables sur le site internet de l'OEB

**Description**

La présente invention concerne un polypeptide du type comprenant un anticorps à domaine unique dérivé d'un anticorps à chaîne lourde naturellement dépourvu de chaîne légère (VHH) de camélidé et dirigé spécifiquement contre la chromatine, plus particulièrement contre des protéines chromatiniennes, ainsi qu'une molécule d'acide nucléique codant pour un tel polypeptide. L'invention concerne également l'utilisation de ce polypeptide, en particulier pour la détection de la chromatine et/ou la visualisation en temps réel de la chromatine sans interférer avec la vitesse de prolifération cellulaire.

A l'heure actuelle, la détection en imagerie de la chromatine peut être réalisée par trois grands types de procédés, plus particulièrement :

- les procédés mettant en oeuvre des molécules fluorescentes qui s'intercalent ou se fixent sur l'ADN, telles par exemple que la 4',6'-diamidino-2-phénylindole (DAPI). Pour la plupart, ces molécules nécessitent la fixation et la perméabilisation des cellules. De plus, elles sont souvent classées comme cancérigènes, mutagènes et reprotoxiques (CMR) et elles nécessitent des précautions d'emploi contraignantes ;

- les procédés mettant en oeuvre des anticorps conventionnels dirigés contre les protéines chromatiniennes, typiquement une histone, qui ne peuvent quant à eux être utilisés que sur des cellules fixées et perméabilisées ;

- et les procédés comprenant l'expression ectopique dans l'organisme cible d'une histone fusionnée à une protéine fluorescente. Ces procédés par transgénèse constituent la seule approche permettant de suivre la chromatine en temps réel dans les cellules vivantes.

La présente invention vise à proposer un outil polyvalent qui puisse être mis en oeuvre dans l'ensemble de ces procédés, et qui permette notamment de détecter / visualiser efficacement la chromatine tant *in vitro* qu'*in vivo*, aussi bien sous forme de protéine recombinante, en remplacement des anticorps conventionnels, par exemple dans les techniques d'immunofluorescence ou de Western blot, qu'en imagerie en temps réel des cellules vivantes ou fixées, par application directe non invasive sur le matériel biologique ou par l'expression de l'ADN codant dans lesdites cellules. Un objectif supplémentaire de l'invention est que cet outil puisse être produit facilement et à bas coût, notamment sous forme pure.

A l'origine de l'invention est un projet à visée toute autre, plus particulièrement un projet visant au développement de biomarqueurs d'effets génotoxiques, précisément la phosphorylation de la sérine 139 de l'histone H2AX. Il a été découvert par les présents inventeurs que des polypeptides particuliers, comprenant le domaine VHH d'anticorps monocaténaires à chaîne lourde des camélidés, présentaient la propriété d'interagir spécifiquement avec la chromatine, et permettaient d'atteindre les objectifs que s'est fixés la présente invention. Il a notamment été découvert par les présents inventeurs, de manière surprenante, que ces polypeptides constituaient un outil permettant de détecter efficacement la chromatine, et en particulier : d'une part, sous forme de protéine recombinante en fusion avec un domaine protéique détectable, par immunofluorescence ou Western blot, avec un bruit de fond avantageusement négligeable ; et d'autre part, exprimés dans des cellules cibles, ou après pénétration dans ces cellules cibles, sans en perturber la progression du cycle cellulaire, si bien qu'ils permettent la visualisation en temps réel des chromosomes mitotiques dans les cellules vivantes en division, ceci sans nécessiter de fixation. Il a également été découvert par les présents inventeurs que les polypeptides présentant de telles propriétés particulièrement avantageuses étaient spécifiquement dirigés contre un complexe d'histones H2A et H2B.

Ainsi, il est proposé selon la présente invention un polypeptide comprenant un anticorps à domaine unique dirigé contre la chromatine, cet anticorps comprenant le domaine variable d'un anticorps à chaîne lourde naturellement dépourvu de chaîne légère (VHH) de camélidé capable de se lier spécifiquement à un complexe d'histones H2A et H2B.

Par « capable de se lier spécifiquement à un complexe d'histones H2A et H2B », on entend, de manière classique en elle-même, le fait que l'anticorps n'est apte à se lier aux histones H2A et H2B que sous leur forme hétérodimérique, et non à chacune de ces histones isolées l'une de l'autre, sous quelque autre forme que ce soit.

Dans « complexe d'histones H2A et H2B », on inclut ici les complexes H2A-H2B dans lesquels une des histones H2A ou H2B, ou les deux, a (ont) subi une ou des modifications post-traductionnelles, telles qu'une acétylation, méthylation, phosphorylation, etc. Pour désigner ce complexe, on utilisera indifféremment dans la présente description les expressions « hétérodimère H2A-H2B » et « dimère H2A-H2B ».

Les anticorps à domaine unique sont des anticorps naturels bien connus en eux-mêmes, consistant en des anticorps à chaîne lourde dépourvus de chaînes légères. Leur domaine variable, communément nommé VHH, ou « nanobody », est typiquement formé d'une pluralité de régions, dont une pluralité de régions charpentes conservées, dites FR, et une pluralité de régions hypervariables, déterminant la complémentarité avec l'antigène, dites CDR. Plus précisément, le domaine VHH comprend une première région charpente FR1, une première région hypervariable CDR1, une deuxième région charpente FR2, une deuxième région hypervariable CDR2, une troisième région charpente FR3, une troisième région hypervariable CDR3, et une quatrième région charpente FR4.

Le domaine VHH des anticorps monocaténaires à chaîne lourde des camélidés suffit à reconnaitre l'antigène : ce domaine monomérique et autonome possède des propriétés de liaison à l'antigène similaires à celles des anticorps conventionnels. De petite taille (15 kDa), particulièrement stable et soluble, il présente notamment l'avantage de pouvoir être produit de façon recombinante, en masse, chez la bactérie ou d'autres espèces tant procaryotes qu'eucaryotes, le cas échéant en fusion avec une ou plusieurs protéine(s) fonctionnelle(s) d'intérêt, et de pouvoir ainsi être utilisé comme un anticorps monoclonal pour la détection d'un antigène.

Par « anticorps dérivé d'un anticorps à chaîne lourde naturellement dépourvu de chaîne légère (VHH) de camélidé », on entend dans la présente description aussi bien les VHH de camélidés eux-mêmes, que leurs dérivés, par exemple les VHH humanisés.

Comme exposé ci-dessus, il a maintenant été découvert par les présents inventeurs que des polypeptides comprenant un anticorps comprenant un domaine VHH de camélidé, et capable de se lier spécifiquement à un hétérodimère H2A-H2B, s'avéraient être des outils particulièrement efficaces pour la détection / visualisation de la chromatine, par des techniques d'imagerie cellulaire, western-blot, cytométrie de flux, etc., ainsi que pour le suivi de la chromatine en imagerie cellulaire en temps réel dans des cellules vivantes ou fixées. Dans ce dernier cas, les polypeptides selon l'invention permettent même de visualiser d'éventuels micronoyaux, ainsi que les ponts anaphasiques, caractéristiques d'un défaut de mitose. Les polypeptides selon l'invention s'avèrent également particulièrement avantageux pour la mise en oeuvre d'expériences de caractérisation fonctionnelle, d'inactivation ou de modification chimique de la chromatine.

Plus particulièrement, les polypeptides selon l'invention, reconnaissant spécifiquement la chromatine, peuvent être utilisés comme anticorps en substitution des anticorps conventionnels, notamment en biochimie, par exemple pour la mise en oeuvre des techniques de western-blot classique et overlay, pull down, Enzyme-linked Immunosorbent Assay (ELISA), etc. Par rapport à la technologie déjà disponible des anticorps classiques, qui sont constitués de deux chaînes lourdes et deux chaînes légères, ces polypeptides à domaine VHH s'avèrent plus compétitifs. En effet, d'une part, leur caractère monomérique permet de facilement les exprimer génétiquement, contrairement aux IgG classiques, et d'autre part, leur petite taille leur confère des propriétés de pénétration cellulaire, mais aussi intramoléculaire remarquables comparés aux anticorps conventionnels. Ils peuvent avantageusement être exprimés en bactérie, et donc purifiés à grande échelle et à très bas coût. En outre, il a notamment été observé par les présents inventeurs que ces polypeptides permettaient une détection efficace de la chromatine, avec un bruit de fond extrêmement faible, ceci chez tous les eucaryotes en immunofluorescence.

De plus, ces polypeptides peuvent être exprimés directement en cellules et ils permettent donc le suivi en temps réel des structures chromatiniennes, par exemple pour suivre les mitoses sur cellules vivantes, tant chez les eucaryotes inférieurs que chez les eucaryotes supérieurs. Un des avantages principaux par rapport aux procédés basés sur l'expression ectopique d'une histone étiquetée dans la cellule, tels que proposés par l'art antérieur, est le fait qu'ils ne modifient pas la stoechiométrie des cibles. Ils permettent en outre une bonne visualisation de la chromatine, et ne perturbent avantageusement pas le cycle cellulaire.

Sur cellules fixées, les polypeptides selon l'invention constituent en outre une alternative avantageuse, notamment du point de vue du coût d'obtention et de mise en oeuvre, à l'utilisation d'agents intercalants pour la coloration du noyau, tels que le DAPI ou l'iodure de propidium. En outre, ils peuvent être fusionnés simultanément à différents peptides/protéines fonctionnels d'intérêt, ce qui permet de passer d'une seule condition d'excitation/émission, pour les agents intercalants chimiques, à plusieurs conditions pour les polypeptides conformes à l'invention, élargissant ainsi les possibilités de marquage multiple.

De manière générale, les polypeptides selon l'invention peuvent par exemple être produits par immunisation de camélidés, par exemple de lamas, avec un hétérodimère H2A-H2B comme immunogène, purification des lymphocytes et construction d'une banque de VHH, puis sélection, notamment par la technique dite de phage-display, à partir de cette banque, exprimée sur la capside d'un phage, des séquences codantes de VHH présentant une affinité pour un hétérodimère H2A-H2B. Ces techniques sont classiques en elles-mêmes, et bien connues de l'homme du métier. Elles sont notamment décrites dans la publication de Lee et al. (2007). Les polypeptides selon l'invention peuvent autrement être obtenus par sélection, à partir d'une banque naïve d'un répertoire de camélidé, par la même technique de phage display, des séquences codantes de VHH présentant une affinité pour un hétérodimère H2A-H2B.

La sélection des VHH capables de se lier à un hétérodimère d'histones H2A et H2B peut être réalisée par tout test de liaison à l'antigène classique en lui-même. Peuvent être mises en oeuvre à cet effet les techniques bien connues de l'homme du métier d'immunoblotting, dot blot, ou immunoabsorption enzymatique ELISA (« enzyme-linked immunosorbent assay »), en mettant à profit le fait que le dimère H2A-H2B peut être formé par simple mise en contact des deux protéines, dans des conditions physico-chimiques, notamment de pH et de salinité, appropriées. A cet effet, il peut notamment être utilisé les histones H2B et H2A humaines, de n° d'accession Genbank respectivement NP_733759.1 (GI:24586679) (H2A type 1-A) et NP_003505.1 (GI:4504249) (H2B type 1). La structure des histones H2A et H2B étant très fortement conservée chez l'ensemble des eucaryotes, les anticorps sélectionnés pour leur capacité de liaison à ce dimère d'origine humaine présentent également la capacité de se lier aux dimères H2A-H2B de toute autre espèce eucaryote, ainsi, notamment, qu'aux dimères comprenant un variant de H2A.

Chez l'humain, l'histone H2A fait partie des histones qui présentent le plus de variants connus. En plus du H2A canonique, la famille comprend quatre variants majeurs codés par des gènes paralogues distincts. Les variants ubiquitaires sont l'histone H2AX, qui joue un rôle central dans la réponse cellulaire aux cassures double brin de l'ADN, et l'histone H2AZ, qui est très conservée chez les eucaryotes, et essentielle à la viabilité cellulaire. Les deux autres membres de la famille H2A ont des profils d'expression spécifiques. Il s'agit de macro H2A, qui se trouve sur le chromosome X inactif, et de H2ABbd, qui est testicule- et cerveau-spécifique. Les polypeptides conformes à l'invention lient avec une grande spécificité tant le dimère H2A-H2B, que les autres dimères comprenant un variant de H2A, en particulier H2AX-H2B et H2AZ-H2B.

Des polypeptides particuliers selon l'invention sont tels que l'anticorps à domaine unique dirigé contre la chromatine présente :

- la séquence d'acides aminés de l'une quelconque des séquences SEQ ID N° 1, SEQ ID N° 2, SEQ ID N° 3 ou SEQ ID N° 4,

- une séquence comprenant une ou plusieurs délétion(s), addition(s) ou substitution(s) d'un ou plusieurs acides aminés par rapport à l'une de ces séquences, qui ne modifient pas de manière significative les caractéristiques de liaison de l'anticorps au complexe d'histones H2A et H2B, ledit anticorps présentant dans un test d'immunoadsorption par enzyme liée (ELISA) dirigé contre un complexe d'histones H2A et H2B, une performance d'au moins 50 % par rapport à l'un ou l'autre des anticorps de séquences SEQ ID N° 1 ou SEQ ID N° 2,

- et/ou une partie fonctionnelle d'une des séquences ci-dessus, conservant le(s) site(s) de liaison et le(s) domaine(s) protéique(s) nécessaires à la liaison au complexe d'histones H2A et H2B, présentant dans un test d'immunoadsorption par enzyme liée (ELISA) dirigé contre un complexe d'histones H2A et H2B, une performance d'au moins 50 % par rapport à l'un ou l'autre des anticorps de séquences SEQ ID N° 1 ou SEQ ID N° 2.

Il est du ressort de l'homme du métier de déterminer quelles modifications peuvent être apportées aux séquences ci-dessus SEQ ID N° 1, SEQ ID N° 2, SEQ ID N° 3 ou SEQ ID N° 4, sans altérer de manière significative les propriétés de reconnaissance de l'antigène, à partir de ses connaissances théoriques et/ou de tests expérimentaux, notamment de tests de liaison à l'antigène hétérodimère H2A-H2B, comme indiqué ci-avant, réalisés sur des séquences obtenues par modifications ciblées ou aléatoires des séquences SEQ ID N° 1 à 4 ci-dessus. Les caractéristiques de liaison de l'anticorps au complexe d'histones H2A et H2B, c'est-à-dire l'avidité et la spécificité, sont considérées ici comme non altérées de manière significative lorsque l'anticorps permet par exemple, mis en oeuvre dans la technique d'imagerie en immunofluorescence, en fusion avec un domaine protéique détectable, de visualiser la chromatine avec un faible bruit de fond.

Préférentiellement, les anticorps dont la séquence comprend une ou plusieurs délétion(s), addition(s) ou substitution(s) d'un ou plusieurs acides aminés par rapport à l'une des séquences SEQ ID N° 1, SEQ ID N° 2, SEQ ID N° 3 ou SEQ ID N° 4, et/ou présentant une partie fonctionnelle d'une de ces séquences, conservant le(s) site(s) de liaison et le(s) domaine(s) protéique(s) nécessaires à la liaison au complexe d'histones H2A et H2B, présentent une performance en ELISA d'au moins 70 %, par rapport à l'un ou l'autre des anticorps de séquences SEQ ID N°1 ou SEQ ID N°2.

En particulier, les modifications par rapport aux séquences SEQ ID N°1 à 4 ci-dessus sont préférentiellement réalisées dans les régions hypervariables CDR des VHH.

Entrent par exemple dans le cadre de l'invention des polypeptides obtenus par modifications des séquences SEQ ID N° 1 à 4 par substitution d'acides aminés par des acides aminés de même famille, par exemple d'un résidu basique tel que l'arginine par un autre résidu basique comme un résidu lysine, d'un résidu acide tel que l'aspartate par un autre résidu acide comme le glutamate, d'un résidu polaire comme la sérine par un autre résidu polaire comme la thréonine, d'un résidu aliphatique comme la leucine par un autre résidu aliphatique comme l'isoleucine, etc.

Des polypeptides selon l'invention présentent une séquence homologue de l'une quelconque de ces séquences ci-dessus SEQ ID N° 1, SEQ ID N°2, SEQ ID N° 3 et SEQ ID N°4, avec une identité de séquence de 79,5 % et plus par rapport à l'une au moins de ces séquences.

Selon une caractéristique préférentielle de l'invention, l'anticorps à domaine unique dirigé contre la chromatine est un domaine VHH.

Autrement, cet anticorps à domaine unique dirigé contre la chromatine peut consister en un VHH humanisé, c'est-à-dire comportant un ou plusieurs acides aminés de la séquence consensus humaine en lieu et place d'un ou plusieurs acides aminés typiques des camélidés. Il est du ressort de l'homme du métier de déterminer quels acides aminés peuvent être ainsi substitués sans perdre la capacité de liaison à l'antigène H2A-H2B, comme indiqué ci-avant.

Entrent également dans le cadre de l'invention des polypeptides dont la séquence est modifiée, par rapport aux séquences SEQ ID N° 1 à 4 ci-dessus, de sorte à augmenter l'affinité plus spécifiquement vis-à-vis d'un dimère H2A-H2B dans lequel une ou les deux histones sont modifiées par des modifications post-traductionnelles participant par exemple à la réponse aux stress et plus généralement à l'expression de l'épigénome. De tels polypeptides permettent avantageusement de suivre spécifiquement des modifications post-traductionnelles des histones, comme les anticorps classiques, mais avec la possibilité d'obtenir une diversité générée rapidement par les techniques de biologie moléculaire, des cribles sur des histones modifiées obtenues par purification biochimique et leur caractérisation en spectrométrie de masse. Ils rendent ainsi possible la mise en évidence de patterns différentiels, *in vivo* ou après fixation, obtenus en réponse à divers stress cellulaires (endo- ou exogènes), dans une utilisation en biologie pronostique ou diagnostique.

Préférentiellement, lorsque l'anticorps à domaine unique dirigé contre la chromatine est un domaine VHH, il présente l'une ou plusieurs des caractéristiques suivantes :

- dans sa région hypervariable CDR1, un résidu arginine ou un résidu glycine en position 29 selon la numérotation de Kabat, c'est-à-dire en première position de la région CDR1, et/ou un résidu sérine ou thréonine en position 30 selon la numérotation de Kabat, c'est-à-dire en deuxième position de la région CDR1 ;

- dans sa région hypervariable CDR2, un résidu glycine en position 58 selon la numérotation de Kabat, c'est-à-dire en cinquième position de la région CDR2, et/ou un résidu thréonine ou un résidu sérine en septième position de la région CDR2 ;

- et dans sa région hypervariable CDR3, un résidu asparagine ou un résidu acide aspartique en dernière position du CDR3, et/ou au moins un des acides aminés suivants, aux positions respectives suivantes, ces positions étant également définies selon la numérotation de Kabat :

  - en position 107 selon la numérotation de Kabat, un résidu arginine ;

  - et/ou en position 104 selon la numérotation de Kabat, un résidu glycine ;

  - et/ou en position 105 selon la numérotation de Kabat, un résidu sérine ou un résidu tyrosine ;

  - et/ou en position 110 selon la numérotation de Kabat, un résidu sérine ou un résidu thréonine.

Le polypeptide selon l'invention peut comprendre un ou plusieurs anticorps à domaine unique dirigés contre la chromatine.

Dans des modes de réalisation particuliers de l'invention, le polypeptide comprend une pluralité d'anticorps à domaine unique dirigés contre la chromatine, chacun de ces anticorps comprenant le domaine variable d'un anticorps à chaîne lourde naturellement dépourvu de chaîne légère (VHH) de camélidé capable de se lier spécifiquement à un complexe d'histones H2A et H2B. Le terme « diabody » peut être utilisé pour qualifier un tel polypeptide comprenant deux anticorps à domaine unique dérivés de VHH.

Ces anticorps à domaine unique dirigés contre la chromatine comprenant un VHH peuvent présenter la même séquence peptidique, ou des séquences peptidiques différentes.

De tels polypeptides constituent avantageusement des sondes moins dynamiques que les polypeptides contenant un seul anticorps à domaine unique dirigé contre la chromatine, leur temps de liaison à l'antigène H2A-H2B étant rallongé. Ces sondes s'avèrent particulièrement utiles pour la mise en oeuvre de techniques d'immunofluorescence, notamment car leur liaison à l'antigène résiste particulièrement bien aux lavages. Elles permettent en particulier de visualiser avec une forte sensibilité l'hétérochromatine, malgré la structure très compactée de cette dernière. Elles permettent en outre d'élargir le champ de visualisation de la chromatine, par exemple de visualiser les gènes éteints, ainsi que de différencier différents états de la chromatine.

Le polypeptide selon l'invention peut en outre comprendre une séquence peptidique correspondant à un peptide fonctionnel d'intérêt ou une protéine fonctionnelle d'intérêt, ou une pluralité de telles séquences peptidiques correspondant à une pluralité de peptides / protéines fonctionnels d'intérêt. L'anticorps à domaine unique dirigé contre la chromatine et ledit ou lesdits peptide(s) / protéine(s) fonctionnel(s) d'intérêt peuvent être séparés par espaceur dans la protéine de fusion ainsi formée.

Selon l'invention, les peptides / protéines fonctionnels d'intérêt peuvent être de tout type, en fonction de l'utilisation particulière visée pour le polypeptide. Par exemple, mais non limitativement, il peut s'agir de protéines détectables, de préférence de protéines détectables par fluorescence, luminescence ou phosphorescence, de protéines d'adressage intracellulaire, ou encore de protéines, notamment d'enzymes, dont on souhaite étudier l'effet sur la chromatine ou les histones, ou dont on souhaite qu'elles affectent les marques épigénétiques de la chromatine. Pour l'application des polypeptides selon l'invention pour la détection / visualisation en temps réel de la chromatine, des exemples non limitatifs de telles protéines fonctionnelles d'intérêt sont les fluorophores GFP, YFP, mCherry, l'étiquette HA, SNAP, Flag, etc. Pour l'application à l'étude de la modification de fibre de chromatine ou d'histones, des exemples de protéines fonctionnelles d'intérêt entrant dans la constitution du polypeptide chimérique selon l'invention sont les ubiquitine-ligase E3, les histone acétyl-transférases, les histone méthyltransférases, les biotine ligases, les endonucléases, etc.

Le ou les peptide(s) / protéine(s) fonctionnel(s) d'intérêt peuvent aussi bien être fusionnés à l'anticorps à domaine unique dirigé contre la chromatine à l'extrémité C-terminale, qu'à l'extrémité N-terminale, de ce dernier. Préférentiellement, la fusion est réalisée à l'extrémité C-terminale de l'anticorps.

Le polypeptide selon l'invention peut en outre avantageusement comprendre une séquence peptidique correspondant à un peptide de pénétration cellulaire, dit CPP (pour l'anglais Cell Penetrating Peptide), tel que le peptide trans activateur de la transcription TAT du virus HIV1, ou le peptide Penetratin commercialisé par la société Innovagen (Chiu et al., 2010).

Un polypeptide particulier selon l'invention comprend une séquence peptidique correspondant à un peptide de pénétration dans les cellules, et une séquence peptidique correspondant à une protéine détectable, telle qu'une protéine fluorescente. Ces séquences peptidiques peuvent être fusionnées à la même extrémité, C-terminale ou N-terminale, de l'anticorps à domaine unique dirigé contre la chromatine, ou respectivement à ses deux extrémités opposées. Un tel polypeptide s'avère en particulier tout à fait avantageux pour une application pour le marquage de la chromatine dans des cellules vivantes. Il peut à cet effet être directement appliqué, sous forme de protéine recombinante purifiée, sur la culture cellulaire, de manière non invasive, et permettre ainsi la visualisation en temps réel de la chromatine, sans nécessiter la mise en oeuvre de techniques de transgénèse.

Un autre aspect de l'invention est une molécule d'acide nucléique codant pour un polypeptide répondant à l'une ou plusieurs des caractéristiques ci-avant.

Un vecteur d'expression comprenant une telle molécule d'acide nucléique peut être de tout type connu en lui-même pour une mise en oeuvre en ingénierie génétique, notamment un plasmide, un cosmide, un virus, un bactériophage, contenant les éléments nécessaires pour la transcription et la traduction de la séquence codant pour le polypeptide selon l'invention.

Une cellule hôte comprenant une molécule d'acide nucléique codant pour un polypeptide selon l'invention, et/ou un vecteur d'expression comprenant une telle molécule peut aussi bien être une cellule procaryote, en particulier bactérienne, notamment pour la production en masse du polypeptide conforme à l'invention, qu'une cellule eucaryote, inférieur ou supérieur, par exemple de levure, d'invertébrés ou de mammifères. Des lignées cellulaires peuvent exprimer, de manière stable, inductible ou constitutive, ou bien transitoire, un polypeptide selon l'invention.

Un animal non-humain transgénique exprimant un polypeptide selon l'invention, en particulier un polypeptide comprenant une séquence peptidique correspondant à une protéine détectable, par exemple une protéine détectable par fluorescence, luminescence ou phosphorescence s'avère notamment particulièrement utile pour des applications à visée médicale, notamment :

- dans le domaine de la recherche, pour l'étude des modalités du développement embryonnaire, notamment pour établir où et quand se produisent les divisions cellulaires, comment les cellules bougent les une par rapport aux autres au cours du développement d'un organisme d'intérêt, etc. ;

- dans le domaine de la prévention, pour établir l'innocuité d'éléments chimiques à partir de l'exposition des modèles animaux transgéniques *ad hoc*, éventuellement au cours de leur développement embryonnaire, par suivi en temps réel de défauts de division cellulaire, de défauts de forme du noyau, de défauts de prolifération, etc. ;

- pour l'évaluation de composés d'intérêt thérapeutique.

Un tel animal transgénique peut être obtenu par toute méthode classique en elle-même, notamment par transformation par un vecteur comprenant un transgène codant pour un polypeptide conforme à l'invention. Préférentiellement, il a intégré ce transgène dans son génome.

Cet animal est de préférence choisi parmi les invertébrés, tels que *Caenorhabditis elegans* ou *Drosophila melanogaster,* les vertébrés inférieurs, tels que le poisson zèbre, ou les souris.

En particulier, il a été montré par les présents inventeurs qu'une drosophile transgénique exprimant un polypeptide conforme à la présente invention, comprenant une séquence peptidique correspondant à une protéine détectable par fluorescence, se développe normalement du stade embryonnaire jusqu'au stade adulte. Le polypeptide y marque de manière spécifique la chromatine, et y reste avantageusement associé pendant tout le développement jusqu'au stade adulte.

Selon un autre aspect, la présente invention concerne un kit, trouvant application notamment pour la détection et/ou la visualisation en temps réel de la chromatine, en particulier d'un complexe d'histones H2A et H2B, ou encore pour leur modification, leur purification, et plus généralement pour leur étude. Ce kit comprend un polypeptide répondant à l'une ou plusieurs des caractéristiques ci-avant, et/ou une molécule d'acide nucléique codant pour un tel polypeptide. Il peut contenir un vecteur d'expression comprenant une telle molécule d'acide nucléique et/ou une cellule hôte les comprenant.

Un procédé de production d'un polypeptide répondant à l'une ou plusieurs des caractéristiques ci-avant, notamment en vue de son utilisation en tant qu'anticorps, comprend :

- la culture de cellules hôtes comprenant une molécule d'acide nucléique codant pour un polypeptide selon l'invention, dans des conditions permettant l'expression de ce polypeptide, par exemple des cellules bactériennes, telles qu'*Escherichia coli*, de levure, telles que *Saccharomyces cerevisiae*, de mammifère ou d'insecte,

- et la récupération du polypeptide ainsi produit.

Un tel procédé permet notamment, mis en oeuvre chez *Escherichia coli* par exemple, une production en masse du polypeptide et à faible coût.

Les polypeptides selon l'invention, les molécules d'acide nucléique codant pour ces polypeptides, les vecteurs d'expression comprenant ces molécules d'acide nucléique, et les cellules hôtes comprenant de telles molécules d'acide nucléique et/ou vecteurs d'expression, trouvent de nombreuses applications, tirant profit à la fois des propriétés d'affinité de l'anticorps à domaine unique pour les histones H2A et H2B sous leur forme hétérodimérique, et des propriétés des domaines VHH des anticorps monocaténaires à chaîne lourde des camélidés, propriétés qui combinées permettent avantageusement un ciblage particulièrement efficace de la chromatine, tant *in vitro* qu'*in vivo.*

La présente invention s'inscrit ainsi, de manière générale, dans le cadre de l'utilisation d'un polypeptide répondant à l'une ou plusieurs des caractéristiques ci-avant, et/ou d'une molécule d'acide nucléique codant pour ce polypeptide, et/ou d'un vecteur d'expression comprenant une telle molécule d'acide nucléique, et/ou d'une cellule hôte les comprenant, pour la fixation d'un peptide / protéine fonctionnel d'intérêt à la chromatine, et plus particulièrement à une ou les deux histone(s) de l'hétérodimère H2A-H2B, ou encore pour provoquer une interférence fonctionnelle *in cellulo*, par inhibition de la liaison de ces histones avec un ligand naturel. Dans le premier cas, le type de peptide / protéine fonctionnel d'intérêt est choisi en fonction de l'application particulière visée.

En particulier, selon l'invention, les polypeptides et/ou les molécules d'acide nucléique selon l'invention peuvent avantageusement être utilisés pour la détection ou la visualisation, en temps réel, en imagerie cellulaire, de la chromatine, en particulier d'une histone de l'hétérodimère H2A-H2B.

Par exemple, sous forme de protéines recombinantes, les polypeptides selon l'invention, dans lesquels l'anticorps à domaine unique dirigé contre la chromatine est fusionné à une étiquette peptidique détectable, peuvent être utilisés en remplacement des anticorps conventionnels, pour la mise en oeuvre de techniques de détection par immunofluorescence, sur cellules fixées, ou par western-blot. De manière tout à fait avantageuse, ils permettent alors le marquage de la chromatine en une seule étape.

En immunofluorescence, sur cellules fixées, les polypeptides selon l'invention permettent tout à fait avantageusement de détecter la chromatine avec un bruit de fond négligeable, ceci chez l'ensemble des eucaryotes. Ils permettent notamment l'imagerie des chromosomes mitotiques dans les cellules en division.

Exprimés directement, au moyen d'un vecteur d'expression conforme à l'invention, dans les cellules cibles, qu'elles soient vivantes ou fixées, l'anticorps à domaine unique dirigé contre la chromatine étant en fusion avec une étiquette peptidique détectable, par exemple un fluorophore protéique, ils permettent en outre de réaliser l'imagerie en temps réel de la chromatine. Des expériences d'imagerie en temps réel de cellules eucaryotes ainsi transfectées ont notamment révélé une très nette concentration du fluorophore protéique sur la chromatine en interphase, ce qui permet de visualiser le noyau cellulaire, ainsi notamment que les micronoyaux et les ponts anaphasiques. Ce résultat avantageux est obtenu quel que soit le fluorophore protéique mis en oeuvre, et que la fusion de ce dernier à l'anticorps à domaine unique dirigé contre la chromatine soit réalisée en C-terminal ou N-terminal de ce dernier. Il est ainsi possible, selon l'invention, de suivre en temps réel l'ensemble de la chorégraphie des chromosomes de la prophase à la télophase. De plus, le fait que les cellules exprimant un polypeptide selon l'invention entrent en mitose démontre que ce dernier n'interfère avantageusement pas avec la progression du cycle cellulaire lorsqu'il est exprimé dans la cellule.

Les propriétés des polypeptides selon l'invention peuvent avantageusement être mises à profit pour la réalisation de tests de génotoxicité. Ainsi, la présente invention concerne également l'utilisation des polypeptides et/ou molécules d'acide nucléique selon l'invention pour la visualisation des perturbations du profil mitotique de la chromatine sur cellules vivantes *ex vivo*, notamment la visualisation de l'apparition d'un ou plusieurs micronoyaux, de fragments de noyaux, d'une migration retardée d'un chromosome, de ponts anaphasiques, c'est-à-dire de fibres de chromatine reliant deux cellules filles, etc., en particulier suite à la mise en présence avec une substance dont la génotoxicité doit être évaluée.

En particulier, le test dit des micronoyaux est un test réglementaire mis en oeuvre pour les études de génotoxicité, qui permet de détecter des substances qui engendrent des lésions cytogénétiques, en particulier les substances clastogènes, qui génèrent des cassures de l'ADN, et/ou aneugènes, qui engendrent un nombre anormal de chromosomes dans les cellules filles, après mitose (Kirsch-Volders (1997) ; Ligne directrice de l'OCDE pour les essais de produits chimiques, N°487, Juillet 2010).

Sur cellules fixées et perméabilisées, les polypeptides selon l'invention, pouvant être produits à faible coût, constituent une alternative avantageuse à l'utilisation d'agents intercalants de type DAPI ou d'autres agents colorants, non intercalants, qui sont quant à eux bien plus coûteux et potentiellement cancérigènes.

Lorsqu'il est produit sous forme recombinante, associé chimiquement, ou comprenant, en fusion traductionnelle avec l'anticorps à domaine unique dirigé contre la chromatine, un fluorophore protéique ou une enzyme d'intérêt, et éventuellement un peptide de pénétration cellulaire, les propriétés du polypeptide selon l'invention peuvent avantageusement être mises à profit pour amener le fluorophore protéique ou l'enzyme d'intérêt à la chromatine dans des cellules vivantes. Ceci peut être réalisé par simple mise en contact du polypeptide avec ces cellules, dont il s'ensuit sa pénétration dans les cellules, et son association spontanée à la chromatine. La visualisation ou la modification de la chromatine dans les cellules vivantes peut alors être avantageusement réalisée sans transgénèse, et très facile à mettre en oeuvre.

Une application des polypeptides selon l'invention, dans lesquels l'anticorps à domaine unique dirigé contre la chromatine est fusionné à une protéine détectable par fluorescence, luminescence ou phosphorescence, et le cas échéant avec un peptide de pénétration cellulaire, et produits sous forme recombinante, est ainsi leur utilisation pour la visualisation en temps réel de la chromatine dans les cellules vivantes, par simple addition du polypeptide dans le milieu de culture cellulaire.

Le polypeptide selon l'invention, et plus particulièrement une lignée cellulaire transgénique stable qui l'exprime, ou des cellules vivantes dans lesquelles il aurait pénétré, peuvent également être utilisés pour l'étude du cycle cellulaire, de la localisation du noyau, de la morphologie du noyau, ceci sans nécessiter de fixation ou de marquage par immunofluorescence.

Un polypeptide répondant à l'une ou plusieurs des caractéristiques ci-avant, et/ou une molécule d'acide nucléique codant pour un tel polypeptide, et/ou un vecteur d'expression comprenant une telle molécule d'acide nucléique, et/ou une cellule hôte les comprenant, peuvent être utilisés pour la modification de fibres de chromatine *in vitro* ou *ex vivo,* sur cellules fixées ou sur cellules vivantes en culture. Préférentiellement, l'anticorps à domaine unique dirigé contre la chromatine y est alors en fusion avec une enzyme dont l'effet sur la chromatine veut être étudié. L'invention trouve alors tout à fait avantageusement de nombreuses applications, par exemple pour la modification de l'état global de la chromatine et donc l'altération des réponses cellulaires, après différents traitements ou pour le criblage de candidats à visée thérapeutique.

Sous forme recombinante, le polypeptide selon l'invention peut autrement être utilisé pour la purification de la chromatine, et en particulier du dimère H2A-H2B, par exemple par une technique d'immunoprécipitation.

Le polypeptide selon l'invention trouve nombre d'autres applications. En particulier, en biochimie, sous forme recombinante, fusionné à une étiquette appropriée, telle qu'une étiquette GST (glutathion-S-transférase) ou CBD (domaine de liaison à la chitine) par exemple, le polypeptide selon l'invention peut être immobilisé sur un support solide, notamment sous forme de billes magnétiques, billes de sépharose, etc. La matrice d'affinité ainsi générée peut alors être utilisée pour purifier la chromatine, immobiliser des nucléosomes, purifier l'octamère d'histones, ou encore l'hétérodimère H2A-H2B, etc.

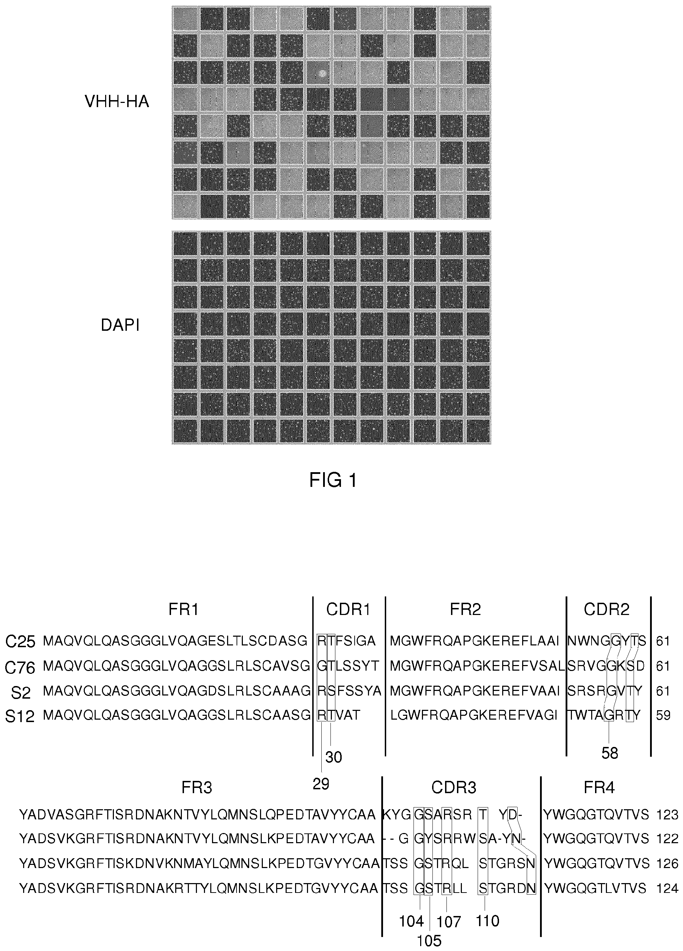

Les caractéristiques et avantages de l'invention apparaîtront plus clairement à la lumière des exemples ci-après, fournis à simple titre illustratif et nullement limitatifs de l'invention, avec l'appui des figures 1 à 26, dans lesquelles :

- la figure 1 montre, pour 96 VHH-HA issus d'une sous-banque de VHH sélectionnée par phage display, les images obtenues par microscopie après immunofluorescence sur cellules fixées (image du haut) et le signal DAPI (image du bas) ;

- la figure 2 montre les structures primaires de quatre anticorps à domaine unique dirigé contre la chromatine selon l'invention, nommés respectivement S2 (de séquence SEQ ID N°1), S12 (de séquence SEQ ID N°2), C25 (de séquence SEQ ID N°3) et C76 (de séquence SEQ ID N°4) ; sur cette figure les différentes régions conservées (FR1, FR2, FR3 et FR4) et hypervariables (CDR1, CDR2, CDR3) de ces anticorps sont alignées ;

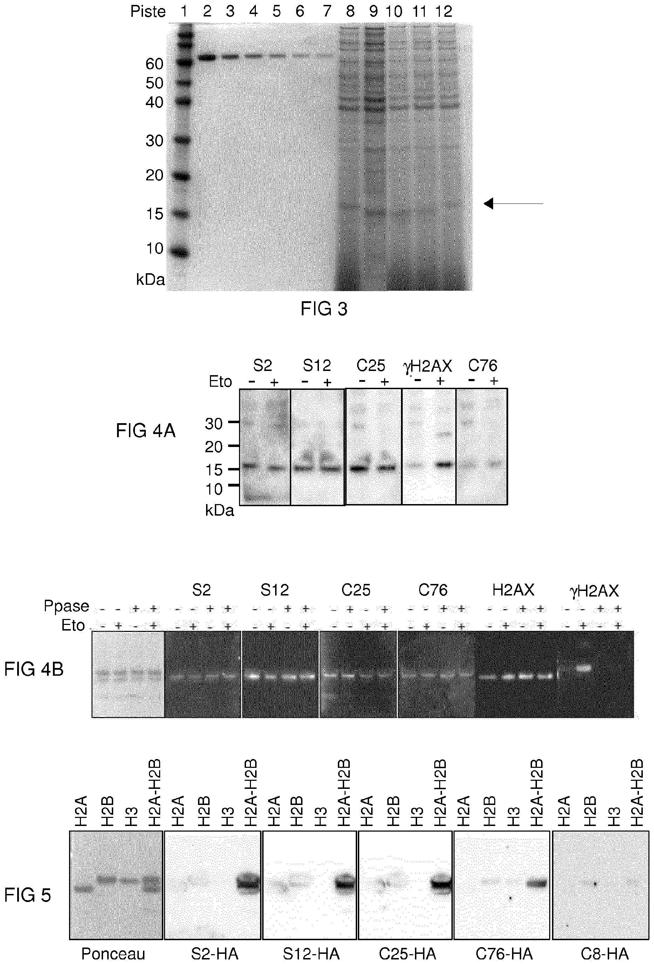

- la figure 3 représente une photographie d'un gel de polyamide dénaturant coloré au bleu de Coomassie, après migration : piste 1, d'un marqueur de poids moléculaire ; pistes 2 à 7, d'une solution contenant respectivement 1000, 500, 250, 125, 62 et 31 ng de BSA ; pistes 8 à 11, de 8 $\mu$l d'un milieu de culture bactérienne dans lequel ont été sécrétés les polypeptides conformes à l'invention respectivement S2-HA, S12-HA, C25-HA, C76-HA ; piste 12, de 8 $\mu$l d'un milieu de culture bactérienne dans lequel a été sécrété le polypeptide comparatif C8-HA ; la flèche indique la bande correspondant à ces polypeptides ;

- les figures 4A et 4B montrent les résultats de tests d'immunoblotting réalisés sur des extraits protéiques de cellules humaines, au moyen respectivement de polypeptides conformes à l'invention S2-HA, S12-HA, C25-HA, C76-HA, et d'anticorps monoclonaux anti-H2AX et anti-yH2AX ; figure 4A, pour des extraits protéiques totaux de cellules humaines HCT116, traitées (« Eto+ ») ou non (« Eto- ») avec l'agent génotoxique étoposide, le marqueur de poids moléculaire étant représenté à gauche sur la figure ; figure 4B, pour les histones totales, obtenues par extraction acide à partir de cellules humaines HT1080 (traitées (« Eto+ ») ou non (« Eto- ») avec l'agent génotoxique étoposide), puis soumises (« Ppase+ ») ou non (« Ppase- ») à déphosphorylation, l'image de gauche correspondant à une membrane colorée au rouge Ponceau ;

- la figure 5 montre les résultats de tests d'immunoblotting réalisés sur des solutions d'histones pures H2A, H2B, H3 et d'un mélange H2A-H2B, au moyen respectivement de polypeptides conformes à l'invention S2-HA, S12-HA, C25-HA, C76-HA, et du polypeptide comparatif C8-HA ; l'image de gauche correspond à une membrane colorée au rouge Ponceau avant incubation avec les polypeptides ;

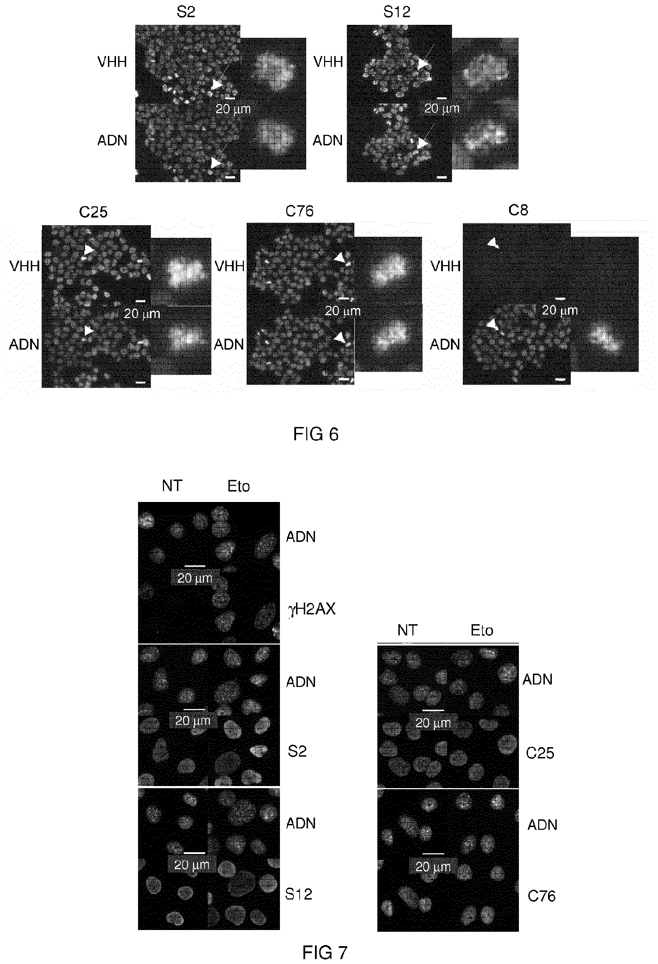

- la figure 6 montre les images de microscopie en fluorescence de cellules humaines HCT116 fixées et perméabilisées traitées en immunofluorescence, montrant le signal VHH (image en haut à gauche) et le signal DAPI (image en bas à gauche), pour des polypeptides selon l'invention (S2-HA, S12-HA, C25-HA et C76-HA) et pour un polypeptide comparatif C8-HA ; pour chaque image, l'image de droite associée montre un agrandissement d'une cellule en mitose indiquée par la flèche blanche sur l'image de gauche ;

- la figure 7 montre des images de microscopie en fluorescence de fibroblastes embryonnaires murins sauvages, sur lesquels des dommages à l'ADN ont été induits (« Eto ») ou non (« NT »), traités en immunofluorescence avec des polypeptides selon l'invention (S2-HA, S12-HA, C25-HA et C76-HA), et un anticorps anti-γH2AX (contrôle) ; pour chacun, sur l'image du haut, « ADN » représente le signal émis par le DAPI ;

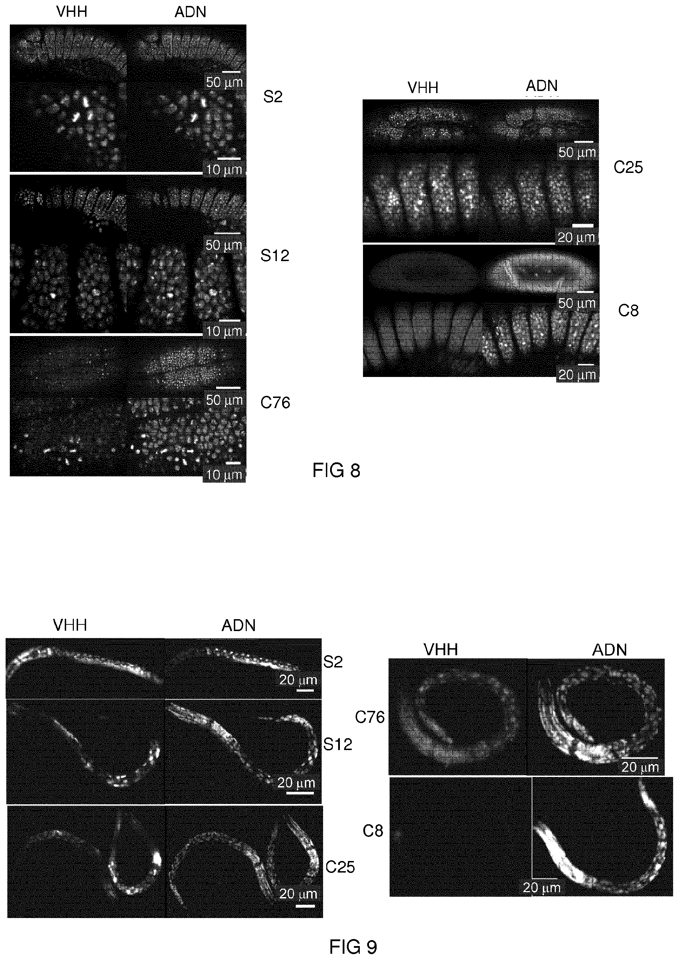

- la figure 8 montre des images de microscopie confocale en fluorescence d'embryons de *Drosophila melanogaster,* fixés et traités en immunofluorescence avec des polypeptides selon l'invention (S2-HA, S12-HA, C76-HA et C25-HA) et un polypeptide comparatif C8-HA, montrant pour chacun de ces polypeptides le signal VHH (image de gauche) et le signal ADN révélé par une coloration à l'iodure de propidium (image de droite) ;

- la figure 9 montre des images de microscopie confocale en fluorescence d'embryons de *Caenorhabditis elegans,* fixés et traités en immunofluorescence avec des polypeptides conformes à l'invention S2-HA, S12-HA, C25-HA et C76-HA, et un polypeptide comparatif C8-HA, montrant pour chacun de ces polypeptides le signal VHH (image de gauche) et le signal ADN révélé par le DAPI (image de droite), chaque ligne présentant deux acquisitions du même champ ;

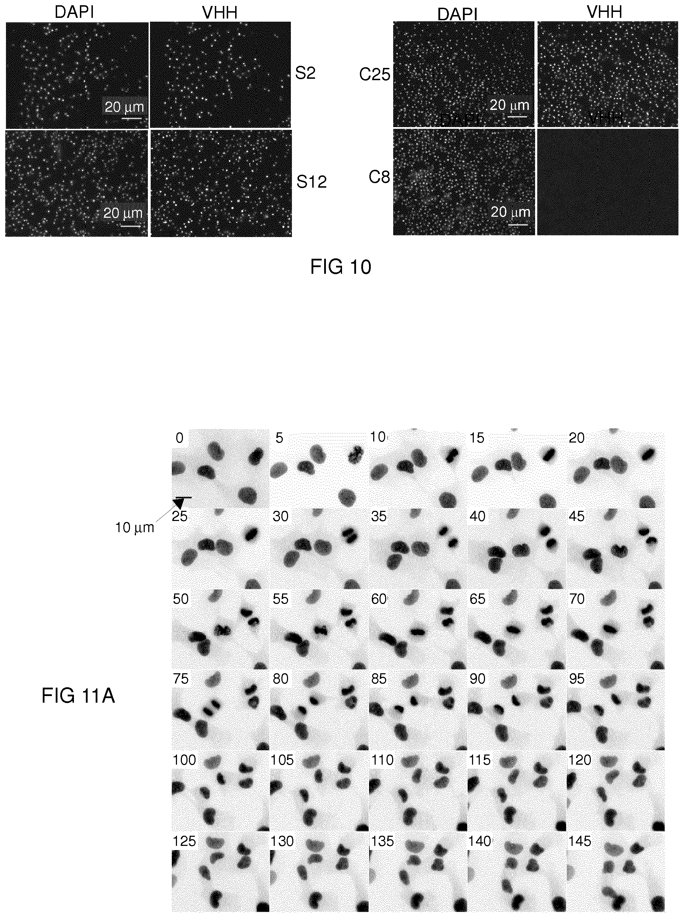

- la figure 10 montre des images de microscopie en fluorescence de cellules de *Saccharomyces cerevisiae* traitées en immunofluorescence avec des polypeptides conformes à l'invention S2-HA, S12-HA et C25-HA, et un polypeptide comparatif C8-HA, montrant pour chacun de ces polypeptides le signal VHH (image de droite) et le signal ADN révélé par le DAPI (image de gauche);

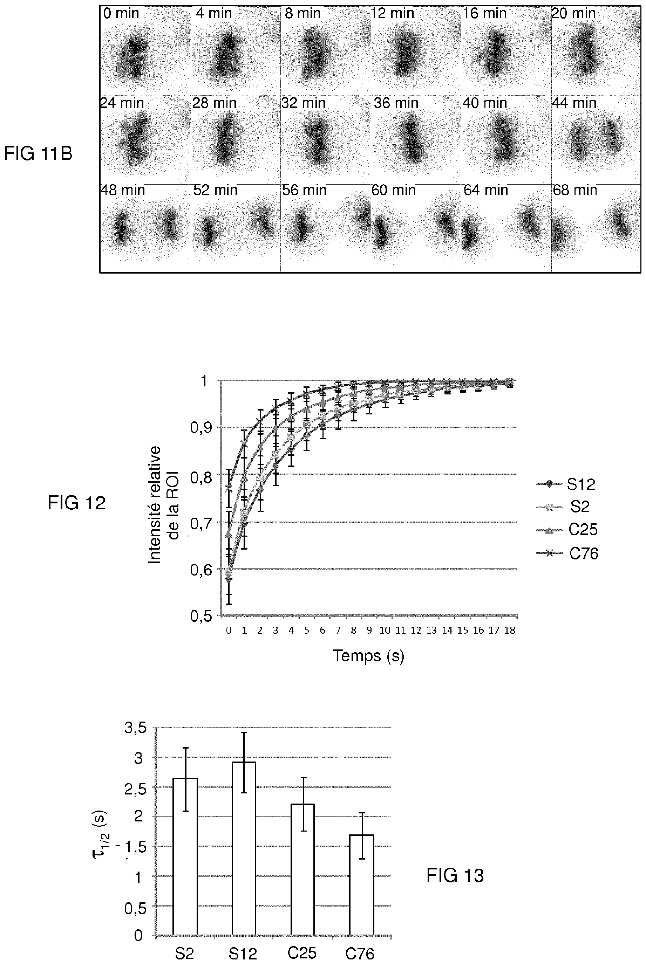

- les figures 11A et 11B montrent des images de microscopie temps réel de la chromatine pendant la progression du cycle cellulaire à travers la mitose ; figure 11A, images de microscopie champ large et temps réel de cellules HT1080 vivantes exprimant de manière stable le polypeptide conforme à l'invention S12-GFP, une pile d'images résultant d'une fréquence d'acquisition de 5 min étant présentée en éclaté avec inversion de l'échelle de gris ; figure 11B, images de microscopie confocale en fluorescence des chromosomes d'une cellule mitotique exprimant S12-GFP, qui progresse de la prométaphase vers la télophase, la fréquence des acquisitions présentées sur l'éclaté étant de 4 min ;

- la figure 12 montre les courbes représentant, pour chacun des polypeptides conformes à l'invention S2-GFP, S12-GFP, C25-GFP et C76-GFP, en fonction du temps, la moyenne (pour 10 mesures indépendantes) des valeurs d'intensité relative de fluorescence dans une région discrète (ROI) du noyau de cellules HT1080 exprimant le polypeptide après photo-blanchiment par une illumination Laser ;

- la figure 13 représente un histogramme présentant, pour chacun des polypeptides conformes à l'invention S2-GFP, S12-GFP, C25-GFP et C76-GFP, la moyenne (pour 10 mesures indépendantes) des valeurs du temps de demi-retour à la fluorescence $\tau_{1/2}$, dans une région discrète du noyau de cellules HT1080 exprimant le polypeptide après photo-blanchiment par une illumination Laser, ces valeurs étant déduites de l'ajustement des courbes constituées à partir des mesures relatives d'intensité en fonction du temps à la fonction $I(t)=I_E - I_1 \times \exp(-t/\tau)$ ;

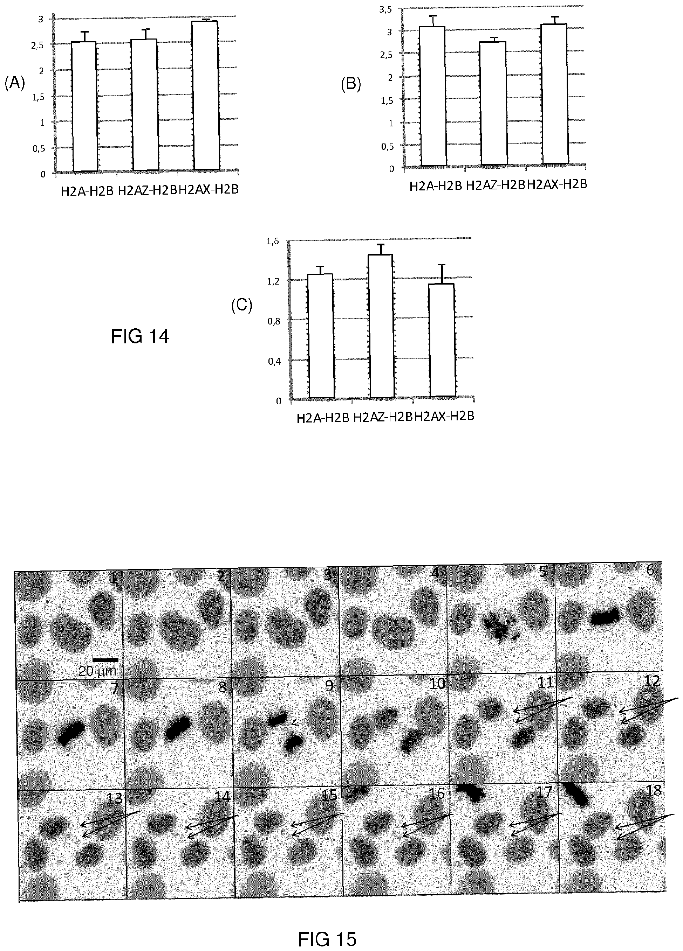

- la figure 14 montre des graphiques représentant la densité optique à 450 nm mesurée par un test ELISA de liaison aux dimères humains respectivement H2A-H2B, H2AX-H2B et H2AZ-H2B, graphique (A), pour le polypeptide confirme à l'invention S2 ; graphique (B), pour le polypeptide confirme à l'invention S12 ; graphique (C), pour un anticorps monoclonal anti-H2B du commerce ;

- la figure 15 montre une pile d'images de microscopie champ large et temps réel résultant d'une fréquence d'acquisition de 10 min, de la chromatine de cellules HT1080 vivantes exprimant de manière stable le polypeptide conforme à l'invention S12-GFP pendant la progression du cycle cellulaire à travers la mitose, permettant de visualiser des micronoyaux (flèches pleines) et un pont anaphasique (flèche en pointillés) ;

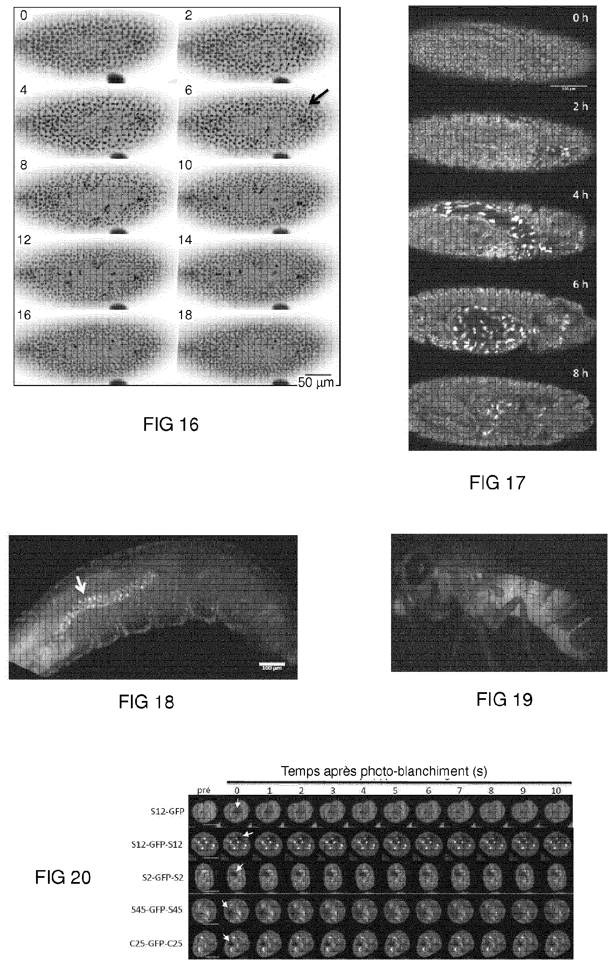

- la figure 16 montre des images de microscopie en temps réel prises toutes les 2 min d'un embryon de *Drosophila melanogaster* transgénique exprimant le polypeptide conforme à l'invention S12-HA-GFP au stade blastoderme syncytial, qui effectue un cycle de division cellulaire ; sur l'image au temps '6 min', la flèche indique des figures d'anaphase ;

- la figure 17 montre cinq images prises à 2 h d'intervalle les unes des autres, provenant d'une expérience de microscopie en temps réel d'une durée totale de 15 h, avec une fréquence d'acquisition de 2 min, de l'embryon de la figure 16 ;

- la figure 18 montre une image de microscopie en épi-fluorescence d'une larve issue d'un embryon fluorescent de la figure 16, la flèche indiquant les noyaux géants des glandes salivaires ;

- la figure 19 montre une image acquise par microscopie en épifluorescence d'un adulte issu de la métamorphose de la larve de la figure 18 ;

- la figure 20 présente des images de microscopie confocale acquise lors d'expériences de FRAP réalisées sur des cellules humaines HCT116 exprimant respectivement les polypeptides conformes à l'invention S2-GFP-S2, S12-GFP-S12, C25-GFP-C25, S45-GFP-S45 ou S12-GFP, à différents temps après blanchiment ;

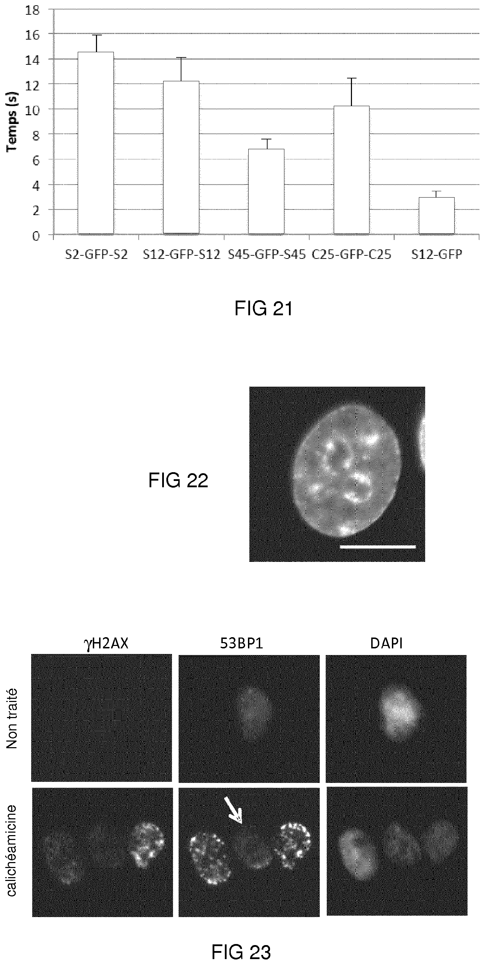

- la figure 21 représente un graphe montrant le temps de demi recouvrement de la fluorescence ($T_{1/2}$) après photo-blanchiment, pour les cinq types de cellules de la figure 20 ;

- la figure 22 montre une image de microscopie confocale d'une cellule humaine HCT116 exprimant le polypeptide conforme à l'invention S12-GFP-S12 ;

- la figure 23 montre les images de fluorescence obtenues, pour une détection respectivement par les anticorps anti-γH2AX, anti-53BP1 et par l'agent intercalant DAPI, pour des cellules humaines HT1080 respectivement non traitées et traitées par la calichéamicine ;

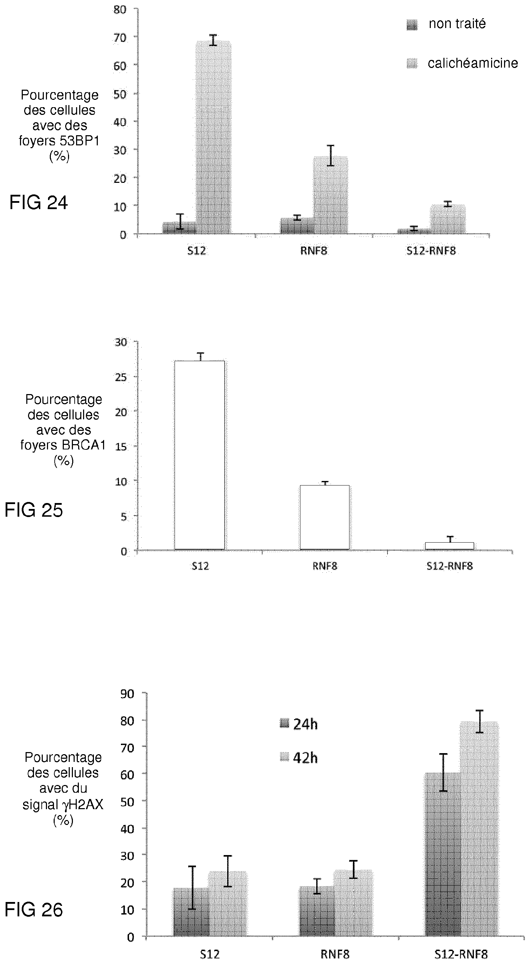

- la figure 24 représente un graphe montrant le pourcentage de cellules humaines HT1080, exprimant soit le polypeptide conforme à l'invention S12-mCherry, soit l'enzyme RNF8-mCherry, soit le polypeptide conforme à l'invention S12-mCherry-RNF8, présentant en immunofluorescence un marquage en foyer détecté par l'anticorps anti-53BP1, caractéristiques de dommages à l'ADN, avec ou sans traitement avec la calichéamicine ;

- la figure 25 représente un graphe montrant le pourcentage de cellules humaines HT1080, exprimant soit le polypeptide conforme à l'invention S12-mCherry, soit l'enzyme RNF8-mCherry, soit le polypeptide conforme à l'invention S12-mCherry-RNF8, présentant en immunofluorescence un marquage en foyer détecté par l'anticorps anti-BRCA1 ;

- et la figure 26 représente un graphe montrant le pourcentage de cellules humaines HT1080, exprimant pendant 24 heures ou 42 heures, soit le polypeptide conforme à l'invention S12-mCherry, soit l'enzyme RNF8-mCherry, soit le polypeptide conforme à l'invention S12-mCherry-RNF8, présentant en immunofluorescence un marquage en foyer détecté par l'anticorps anti-γH2AX, caractéristiques de dommages à l'ADN.

EXPERIENCE 1 - Identification d'anticorps à domaine unique dirigés contre la chromatine selon l'invention

Des anticorps à domaine unique dirigés contre la chromatine selon l'invention ont été identifiés par les présents inventeurs de la façon suivante.

Construction d'une banque de VHH

Une banque d'expression dédiée au phage display, contenant les séquences codantes du répertoire immun VHH d'un lama (*Lama glama*), a été construite. Les ARN totaux des lymphocytes d'un lama immunisé par un phospho-peptide H2AX synthétique γH2AX de séquence SEQ ID N°6 (KKATQAS$^{PO4}$QEY) ont été isolés. Ils ont été convertis en ADNc par l'action d'une reverse transcriptase (Superscript® II First Strand synthesis, Invitrogen), après hybridation d'amorces aléatoires.

Les ADNc ainsi générés ont servi de matrice pour une réaction de polymérisation en chaîne (PCR), en utilisant le couple d'amorces :

CALL01 : 5' GTCCTGGCTGCTCTTCTACAAGG 3' (SEQ ID N°7)

CALL02 : 5' GGTACGTGCTGTTGAACTGTTCC 3' (SEQ ID N°8)

permettant l'amplification de la partie variable des chaînes lourdes des immunoglobulines en deux produits PCR de taille différente, correspondant respectivement aux VH et VHH.

Les séquences codantes des VHH ont été séparées de celles des VH par une électrophorèse en gel d'agarose. Après extraction du gel d'agarose, les ADNc des VHH ont servi de matériel de départ pour deux PCR nichées successives, visant à ajouter dans la bonne phase de lecture les sites de clonage PstI en 5' et NotI en 3'.

Après digestion par les enzymes de restriction PstI et NotI, l'ADN a été purifié puis inclus par une réaction de ligation, au moyen d'une T4 DNA ligase, dans le vecteur pHEN4 décrit dans la publication d'Arbabi Ghahroudi et al. (1997), digéré par les mêmes enzymes.

Le produit de ligation a été introduit dans des bactéries TG1 (sup E) par électroporation, et les cellules transformées ont été étalées sur 96 boîtes (23 x 23 cm) contenant du milieu LB agar additionné d'ampicilline.

L'ensemble des colonies bactériennes obtenues a été récolté et la diversité de la banque ainsi générée a été estimée à $6,2.10^7$.

Établissement de lignées cellulaires exprimant de manière stable l'histone H2AX fusionnée aux étiquettes Strep ou Chitin Binding Domain (CBD)

Afin de pouvoir immobiliser l'histone H2AX sur un support macroscopique solide (billes magnétiques), des vecteurs permettant d'établir l'expression stable de deux formes étiquetées de H2AX ont été construits.

La séquence codante de H2AX a été amplifiée par PCR avec les amorces suivantes :

For 5' ACGGTACCTCGGGCCGCGGCAAG 3' (SEQ ID N°9)

Rev 5' CGGATCCCTATTAGTACTCCTGGGAGGC 3' (SEQ ID N° 10)

Le fragment généré, digéré par les enzymes de restriction KpnI et BamHI, a ensuite été cloné aux sites KpnI - BamHI dans deux vecteurs d'expression. Le vecteur pCBD-H2AX obtenu, dérivé du pTYB21 (New England Biolabs), permet l'expression de H2AX en fusion N-terminale avec le domaine de liaison à la chitine. Le vecteur p2xStrep-H2AX obtenu, dérivé du Strep-tag II (iba-lifesciences), permet l'expression de H2AX en fusion N-terminale avec le double tag Strep (2xStrep), de séquence SEQ ID N° 11 (WSHPQFEKGGGSGGGSGGGGSAWSHPQFEK) où la séquence WSHPQFEK représente l'étiquette Strep.

La lignée cellulaire permettant l'expression stable de CBD-H2AX a été générée par transfection du vecteur pCBD H2AX dans des cellules HT1080 (fibrosarcome humain) par l'agent de transfection jetPEI® (Poly-Plus-transfection), puis sélection des intégrants stables par la généticine (G418).

L'établissement de cellules HT1080 exprimant de manière stable 2xStrep-H2AX a été réalisé par transduction lentivirale. Pour cela, la séquence codante de la fusion a été sous clonée par les sites NheI-BamHI dans le vecteur navette pTRIP-CMV XNCA (Sirven et al., 2001). Le vecteur pTRIP 2xStrep-H2AX résultant a permis de générer des particules lentivirales, utilisées pour transduire des cellules HT1080.

Extraction de l'histone H2AX étiquetée de la chromatine et préparation des billes magnétiques

Afin d'induire la phosphorylation de H2AX sur la serine 139, les cellules HT1080 exprimant CBD-H2AX ou 2xStrep-H2AX précédemment décrites ont été traitées avec l'agent radiomimétique calichéamicine à 4 nM final. Les histones ont par la suite été isolées par extraction selon le protocole décrit par Shechter et al. (2007).

Succinctement, les noyaux ont été préparés par un traitement hypotonique combiné avec le détergent non ionique NP-40. Ils ont été lysés dans un tampon haut sel (2,5 M NaCl) qui permet de conserver les modifications post-traductionnelles et l'interaction entre H2A et H2B, puis l'ADN génomique a été fragmenté par sonication. Ce matériel a servi à charger des billes magnétiques de chitine (New England Biolabs) ou Strep-Tactin® (iba-lifesciences).

Pour charger les billes de chitine, les billes magnétiques de chitine ont été lavées avec le tampon de lavage (Tris pH 8 20 mM, EDTA 1 mM, triton X-100 0,05 %) contenant du chlorure de sodium (NaCl) à 500 mM, et incubées dans le tampon de blocage (tampon de lavage + 5 % BSA (albumine de sérum bovin)) pendant 40 min à 4 °C sous agitation. Les billes ainsi bloquées ont été lavées 2 fois avec du tampon de lavage 2 M NaCl, reprises dans 1 mL de tampon de lavage 2 M NaCl, puis incubées avec les histones purifiées pendant la nuit à 4 °C sous agitation. Les billes ont alors été lavées extensivement (10 fois avec du tampon de lavage 2 M NaCl), le dernier lavage se faisant en tampon de lavage 500 mM NaCl. Les billes magnétiques Strep-Tactin® (iba-lifesciences) ont été chargées par incubation des billes dans un extrait haut sel obtenu sur des cellules HT1080::2xStrep-H2AX, puis le matériel a été lavé dans un tampon phosphate 300 mM NaCl.

Sélection des clones par phage display et génération des sous-banques

Cette étape a initialement visé à sélectionner des séquences codantes de VHH ayant de l'affinité pour l'histone γH2AX étiquetée, isolée de la chromatine de cellules humaines transgéniques. Le processus de sélection, décrit dans la publication de Lee et al. (2007), a impliqué trois phases distinctes. Dans un premier temps, une première sous-banque a été générée par une sélection phage display contre CBD-yH2AX sur les billes de chitine, avec la BSA comme agent bloquant. Cette première sous-banque VHH a ensuite servi de base dans deux sélections phage display distinctes, suivant le protocole détaillé décrit ci-après : l'une de nouveau contre CBD-yH2AX, avec la β-caséine comme agent bloquant pour générer la sous-banque dite C, l'autre contre 2xStrep-γH2AX, pour générer la sous-banque dite S.

Amplification de la banque et génération des phages

Un aliquot bactérien de 1 mL (représentant 100 fois la diversité de la banque) a été amplifié dans 30 ml de milieu TY2X (20 min, 37 °C, 250 rpm) jusqu'à obtenir une DO à 600 nm de 0,25, avant d'être dilué dans 100 mL de TY2X additionné d'ampicilline à 100 μg/mL et de glucose à 1 %. La culture a été incubée 2 h, à 37 °C et 250 rpm, jusqu'à obtenir une DO à 600 nm de 0,5.

Les bactéries sont alors infectées avec $10^{12}$ phages helper, correspondant à une multiplicité d'infection (MOI) de 40, et incubées 1 h à 37 °C, sans agitation. Après une incubation de 20 min à 37 °C à 230 rpm, la culture est centrifugée 10 min à 2000 rpm. Le culot bactérien, resuspendu dans 300 ml de TY2X additionné d'ampicilline à 100 μg/mL et de kanamycine à 75 μg/mL, est incubé pendant la nuit à 30 °C à 130 rpm. La culture est centrifugée 20 min à 5000 g, et le surnageant contenant les phages est récupéré. Ces derniers sont précipités 1h à froid au moyen de PEG8000 4 % 0,5 M NaCl, centrifugés 30 min à 4000 rpm à 4 °C puis resuspendus dans une solution de PBS 1X stérile, à raison d'environ $10^{12}$ phages/mL. La suspension est incubée avec 1 mL de tampon de blocage (tampon de lavage (Tris pH 8 20 mM, EDTA 1 mM, triton X-100 0,05 %) additionné de NaCl à 500 mM et de 5 % BSA) et 40 μl de billes, préalablement équilibrées en tampon de lavage 500 mM NaCl, pendant 40 minutes à 4 °C sous agitation. Les phages ne s'étant pas fixés aux billes de manière aspécifique sont récupérés dans le surnageant.

Stratégie de purification des phages par affinité

Les phages sont incubés avec les billes de chitine CBD-yH2AX pendant 3 h à 4 °C sous agitation. Les billes sont lavées extensivement (10 lavages (tampon de lavage à 500 mM NaCl), les deux derniers lavages s'effectuant sans Triton@ X-100), puis incubées dans du tampon de lavage additionné de trypsine 1,25 μg/ml et sans Triton®, pendant 30 min à température ambiante et sous agitation, afin d'inactiver les phages helper. Les billes sont alors incubées dans une solution TEA 2,8 % pendant 7 min sous agitation. Après ajout de Tris pH 7,4 à 1,5 M, sous agitation douce, le surnageant contenant les phages élués est récupéré.

Les phages élués sont utilisés pour infecter des bactéries TG1, cultivées en milieu LB additionné de glucose 1 % jusqu'à une DO à 600 nm de 0,35, à 30 °C sans agitation pendant 1 h (MOI de 40). De plus, les bactéries TG1 sont ajoutées sur les billes contenant les phages non élués, incubées à 30 °C sans agitation pendant 1 h. Les bactéries TG1 sont rassemblées et étalées sur milieu TY2X agar additionné d'ampicilline à 100 μg/mL et de glucose à 0,5 %, et incubées 1 nuit à 30 °C. Les colonies sont récoltées en milieu LB liquide, et la suspension de bactéries centrifugée pendant 10 min à 2000 rpm et 4 °C. Le culot de bactéries est resuspendu afin de constituer la sous-banque du tour suivant de phage display. L'ajout de 50 % glycérol a permis sa conservation à -80 °C.

Le deuxième tour de phage display a été réalisé de façon identique à celui décrit précédemment, en changeant cependant l'étiquette de la protéine immobilisée sur la colonne (étiquette 2xStrep), les billes d'affinité (billes Strep-Tactin®, iba-lifesciences) et l'agent bloquant (β-caséine, 1 mg/ml au final). La suspension bactérienne obtenue à la fin de ce deuxième tour de phage-display représente la sous-banque S (dont sont issus les clones S2 et S12 décrits ci-après).

Parallèlement, une deuxième stratégie de purification des phages par affinité a été développée. Cette stratégie est comparable à celle décrite précédemment, excepté les modifications suivantes. Le premier cycle de phage-display a été réalisé sur une colonne Strep-Tactin® préparée avec la protéine de fusion 2xStrep-γH2AX et la BSA comme agent bloquant. Le second cycle de phage-display a été réalisé sur billes de chitine avec la protéine fusion CBD-yH2AX et la β-caséine comme agent bloquant. Enfin, les protéines de fusion ont été obtenues à partir de l'extraction d'histone réalisée sur les lignées stables décrites précédemment, selon le protocole d'extraction à forte concentration en sel (Shechter et al. (2007)), suivi d'une sonication afin de fragmenter la chromatine. Cette deuxième stratégie a permis d'obtenir une sous-banque dite C (dont sont issus les clones C25 et C76 décrits ci-après).

Crible des sous banques S et C et identification de polypeptides selon l'invention

La stratégie retenue pour évaluer les sous-banques générées a consisté à produire (dans le périplasme) de manière clonale les VHH de la sous-banque, à partir de clones bactériens piqués au hasard, puis d'utiliser le milieu bactérien clonal dans lequel le VHH-HA est sécrété en immunofluorescence sur des cellules fixées, et enfin d'analyser en microscopie à fluorescence la structure intracellulaire reconnue par le clone VHH.

Une plaque 96 puits (Deep well) contenant du milieu de culture bactérien (2TY additionné d'ampicilline 100 μg/ml) est ensemencée de manière clonale avec des clones d'une sous-banque piqués au hasard. La plaque est incubée à 37 °C sous agitation pendant 5 h. La $DO_{600}$ atteint environ 0,4. Puis de l'isopropyl β-D-1-thiogalactopyranoside (IPTG) est ajouté dans chaque puits (pour atteindre une concentration finale de 1 mM), de manière à induire la production des VHH. La température de culture est abaissée à 28 °C et la production est effectuée sur la nuit.

Parallèlement, une plaque 96 puits dédiée à l'imagerie cellulaire (Imaging Plate BD Falcon®, coloris noir fond transparent) est ensemencée avec des cellules HT1080 à raison de $2.10^4$ cellules par puits, et placée dans un incubateur à $CO_2$ de culture cellulaire pendant la nuit. La plaque de culture bactérienne est centrifugée (4000 rpm, 15 min) afin de séparer les bactéries de leur milieu et de transférer spécifiquement le surnageant contenant le VHH-HA sécrété. Les cellules en culture dans les puits de la plaque d'imagerie sont traitées à l'étoposide (10 μM) pendant 1 h, pour induire des cassures de l'ADN, fixées au paraformaldéhyde 4 % pendant 15 min et perméabilisées, par un traitement par 0,5 % Triton@ X100 pendant 5 min. 50 μl de milieu bactérien VHH-HA de la plaque fille sont transférés dans une plaque 96 puits, dont les puits contiennent 50 μl d'une dilution au 1/1000 d'un anticorps anti-HA (anti-HA 11 clone 16B12 Covance). Ce mélange est transféré dans la plaque d'imagerie contenant les cellules fixées et perméabilisées. Après incubation (1 h à température ambiante) et un lavage au PBS, l'anti HA est détecté par ajout dans les puits de la plaque de 50 μl d'anticorps anti-souris couplé à l'Alexa Fluor® 488 (dilué au 1/1000 et incubé 1 heure sur les cellules).

Après lavage de l'anticorps secondaire, l'ADN est marqué au DAPI, puis les cellules de chaque puits de la plaque sont imagées à l'aide d'un microscope inversé robotisé (ArrayScan Cellomics HCS reader).

Les images obtenues pour la sous-banque S sont montrées sur la figure 1. Sur cette figure, l'image du haut représente le signal obtenu par immunofluorescence, et l'image du bas représente le signal DAPI. On y observe qu'un marquage chromatinien homogène remarquablement spécifique est observé pour un certain nombre de clones.

L'ADN plasmidique de tous ces clones produisant un VHH marquant la chromatine a été purifié, puis séquencé. Les séquences ont été alignées afin de purger les redondances. L'élimination des redondances des clones chromatiniens a permis de dégager deux VHH de la sous-banque S, nommés respectivement S2 (de séquence SEQ ID N°1) et S12 (de séquence SEQ ID N°2), ainsi que deux VHH de la sous-banque C, nommés respectivement C25 (de séquence SEQ ID N°3) et C76 (de séquence SEQ ID N°4).

Les séquences peptidiques du domaine VHH de chacun de ces clones sont montrées sur la figure 2. Sur cette figure sont indiquées les différentes régions conservées et hypervariables de chaque séquence, ainsi que les résidus conservés particulièrement importants pour la liaison à la chromatine dans le CDR3, en positions respectivement 104, 105, 107 et 110 selon la numérotation de Kabat. Ces acides aminés, ainsi que les autres acides aminés conservés entre les différentes séquences, dans le CDR1, le CDR2 et le CDR3, sont mis en évidence par un encadrement sur cette figure.

A titre d'exemple comparatif négatif pour les tests ci-après, un clone C8, de séquence SEQ ID N°5, ne montrant pas d'affinité pour la chromatine, a également été sélectionné.

EXPERIENCE 2 - Production des polypeptides recombinants chez *Escherichia coli*

Les polypeptides conformes à l'invention S2-HA, S12-HA, C25-HA, et C75-HA, ainsi que le polypeptide comparatif C8, ont été produits chez *E. coli,* de la manière suivante.

Pour chacun, le vecteur d'expression périplasmique bactérien pHEN4-VHH décrit ci-avant dans l'Expérience 1, est utilisé pour transformer la souche *E. coli* BL21 (DE3) (transformation au CaCl2).

Du milieu de culture 2TY contenant 100 μg/mL d'ampicilline et 0,2 % de glucose est inoculé avec une colonie isolée issue de cette transformation, puis soumis à agitation à 37 °C et 220 rpm jusqu'à obtenir une

DO$_{600nm}$ de 0,5. De l'IPTG, à 1 mM final, est alors ajouté à la culture, qui est poursuivie à 28 °C toute la nuit (soit pendant 16 h). La suspension bactérienne est centrifugée à 7000 rpm pendant 10 min, et le milieu, contenant le polypeptide selon l'invention, est récupéré.

Un échantillon de 8 μl de chaque solution ainsi obtenue est soumis à analyse par séparation sur un gel de polyacrylamide dénaturant (SDS-PAGE) et coloration du gel au bleu de Coomassie. Des solutions de BSA à différentes concentrations connues sont soumises aux mêmes opérations, de sorte à évaluer, par comparaison de l'intensité des bandes respectives, la concentration du polypeptide selon l'invention (correspondant à une chimère anticorps à domaine unique dirigé contre la chromatine - étiquette HA) sécrété dans le milieu.

Le gel obtenu après coloration est montré sur la figure 3. On y observe, pour chaque échantillon de milieu de culture, une bande d'environ 15 kDa, correspondant aux polypeptides selon l'invention S2-HA, S12-HA, C25-HA et C75-HA et au polypeptide comparatif C8-HA. On en déduit en outre, pour chacun des polypeptides testés, une concentration en polypeptide (correspondant à la bande indiquée par une flèche sur la figure) d'environ 20 μg/ml.

Ces solutions peuvent être directement utilisées pour l'immunofluorescence ou l'immunoblotting.

EXPERIENCE 3 - Identification de la cible moléculaire des polypeptides conformes à l'invention S2, S12, C25 et C76

Afin d'identifier la cible de S2, S12, C25 et C76, ces polypeptides, produits sous forme recombinante, ont été utilisés dans des expériences d'immunoblotting. Le polypeptide C8 a été utilisé en tant que témoin comparatif.

3.1/ Analyse par immunoblottinq sur des extraits totaux de cellules humaines

Afin de déterminer si les polypeptides objets de la présente invention sont capables de reconnaître leur cible immobilisée sur une membrane de nitrocellulose, et le cas échéant si le patron du signal est modifié par les dommages à l'ADN, et afin de définir la masse moléculaire de la cible à partir de sa mobilité électrophorétique, une expérience d'immunoblotting classique a été menée sur des extraits protéiques totaux préparés à partir de cellules humaines, traitées ou non à l'étoposide.

A cet effet, les protéines cellulaires totales de cellules humaines HCT116, traitées (« Eto+ ») ou non (« Eto- ») avec l'agent génotoxique étoposide, sont extraites par lyse directe des cellules dans le tampon de charge SDS. Le lysat ainsi obtenu est soniqué avant son dépôt sur gel.

Les protéines sont séparées par électrophorèse en gel dénaturant de polyacrylamide (NuPage® Bis Tris, Life technologies), puis transférées sur membrane de nitrocellulose.

Après un blocage dans du TBS-T (Tris Buffered Saline 0,1 % Tween® 20), 5 % lait écrémé, la membrane est incubée pendant 1 h dans une dilution du polypeptide VHH-HA (5 µg/ml) combiné avec un anticorps anti-HA (HA-11 monoclonal souris 16B12 de Covance) dilué à 1 µg/ml dans du TBST, 5 % lait écrémé. Les membranes sont également sondées avec les anticorps monoclonaux anti-H2AX (Epitomics clone EPR895) et anti-γH2AX (Millipore). Après 3 lavages en TBS-T, le blot est incubé avec l'anticorps secondaire anti-souris couplé à la Horse Radish Peroxydase (HRP) (Cell Signaling), puis lavé 3 fois 10 min. La révélation est effectuée avec un substrat ECL (Enhanced Chemi-Luminescence West Dura Thermo scientific). L'émission de photon de la chemiluminescence est capturée par une caméra (G-Box Syngene).

Les images obtenues sont montrées sur la figure 4A. On y observe que les polypeptides conformes à l'invention S2, S12, C25 et C76 reconnaissent tous une bande de mobilité électrophorétique similaire, légèrement au dessus de 15 kDa. La nature des signaux générés par les polypeptides de la présente invention n'est pas modifiée par l'induction de dommages à l'ADN, contrairement à ce qui est observé avec le contrôle anticorps anti-γH2AX. De manière remarquable, la masse moléculaire apparente de γH2AX et celles des espèces reconnues par les polypeptides objets de l'invention sont similaires, suggérant que ces derniers pourraient reconnaître une histone H2A ou H2B.

### 3.2/ Analyse par immunoblotting sur les histones de cellules humaines obtenus par extraction acide

Afin de tester cette hypothèse, mais aussi déterminer si les VHH objets de l'invention reconnaissent un phospho-épitope, les histones ont été préparées à partir de cellules humaines en culture, traitées ou non à l'étoposide, par extraction acide.

A cet effet, les histones sont extraites de cellules cultivées *in vitro* (HT1080), traitées ou non traitées par l'étoposide, par la technique de l'extraction acide décrite dans la publication de Shechter et al. (2007). Succinctement, après trypsination d'une culture adhérente sur plastique, $10^7$ cellules sont lavées au PBS et pré-extraites par re-suspension dans 1 ml de tampon d'extraction (10 mM HEPES pH 7,5, 10 mM KCl, 1,5 mM $MgCl_2$, 0,34 M saccharose, 0,5 % NP40, 10 % glycérol, cocktail d'inhibiteurs des protéases et phosphatases (Thermo)).

Après 10 min d'incubation dans la glace, le matériel insoluble (noyaux) est culotté par centrifugation (10 000 g, 10 min à 4 °C). Les noyaux sont repris dans 400 µl de 0,4 N $H_2SO_4$ et incubés sur roue pendant 2 h. Après centrifugation (16 000 g, 10 min à 4 °C), le surnageant contenant les histones est récupéré et les histones sont précipitées par ajout d'acide tri-chloro acétique (TCA, 33 % final) et incubation sur glace pendant 1 h. Les histones sont culottées par centrifugation à 16 000 g, pendant 10 min à 4 °C, et le culot lavé 2 fois dans de l'acétone refroidie à -20 °C. Après séchage, le culot d'histone est repris dans 100 µl d'eau.

Une partie des histones ainsi obtenues par extraction acide sont, si nécessaire, déphosphorylées par un traitement à la phosphatase alcaline (Roche) selon le protocole fourni par le fournisseur.

Les deux préparations d'histones ainsi générées, traitées ou non par phosphatase, sont séparées par électrophorèse SDS-PAGE et transférées sur membrane de nitrocellulose, selon le protocole décrit ci-avant. Une coloration au rouge Ponceau est réalisée en parallèle.

Les résultats obtenus sont montrés sur la figure 4B. On y observe que les polypeptides S2, S12, C25 et C76 reconnaissent des espèces moléculaires dont les mobilités sont très similaires et légèrement situées au dessus du marqueur moléculaire 15 kDa.

De manière remarquable, la nature des signaux n'est ni modifiée par les dommages à l'ADN, ni par le traitement phosphatase, alors que l'un et l'autre de ces deux traitements modifient largement le signal généré par l'anticorps anti-gamma H2AX (utilisé ici comme le contrôle de déphosphorylation).

Ces résultats montrent que la cible des polypeptides objets de la présente invention est soluble dans l'acide, et n'est pas un phospho-épitope. De plus, la superposition de la colorations au rouge Ponceau, qui permet de visualiser les protéines présentes sur la membrane, avec le signal généré par les VHH S2, S12, C25, et C76, montre que ces derniers reconnaissent des espèces moléculaires se situant entre H3/H2B (qui possèdent une mobilité quasi-identique dans ces conditions d'électrophorèse) et H2A. Ces données restreignent les cibles des VHH conformes à l'invention aux histones H3, H2A, H2B, et permettent d'exclure l'histone H4, ainsi que l'histone H1 en tant que cible moléculaire.

### 3.3/ Analyse par immunoblotting sur des histones purifiées

Afin de caractériser précisément la cible des VHH objets de l'invention, les mêmes expériences d'immunoblotting ont été menées sur les histones individuelles, obtenues par purification par HPLC.

Afin de purifier individuellement chacune des 5 classes d'histones, un protocole de séparation des histones par chromatographie haute pression en phase inverse (RP HPLC), décrit dans l'article de Shechter et al. (2007), a été suivi. Plusieurs milligrammes d'histones solubilisées dans l'eau ont été injectés dans une colonne de phase inverse de type C4 équilibrée avec 5 % d'acétonitrile, 0,1 % d'acide trifluoroacétique (TFA) dans de l'eau. L'élution des histones a été réalisée par un gradient d'acétonitrile (5 - 90 % sur 120 min, avec un débit de 0,8 ml/min). Les fractions obtenues (de 1 mL chacune) ont été lyophilisées, puis reprises dans 100 µl d'eau avant d'être analysées par électrophorèse en gel SDS-PAGE. Les fractions contenant les histones pures ont été retenues, et ont servi à la caractérisation de la cible des polypeptides objet de l'invention.

A cet effet, les protéines des fractions ainsi obtenues, en particulier des fractions contenant H2A, H2B et H3, ainsi qu'un mélange des fractions H2A et H2B, sont séparées par électrophorèse en gel dénaturant de polyacrylamide (NuPage® Bis Tris, Life technologies), puis transférées sur membrane de nitrocellulose. La révélation est réalisée selon le protocole décrit ci-avant.

Les résultats obtenus sont montrés sur la figure 5. Cette figure montre que S2, S12, C25 et C76 sont capables de générer un signal uniquement sur la piste qui contient le mélange H2A - H2B. Ce signal est précisément localisé à l'interface des deux bandes H2A et H2B, allant dans le sens d'une reconnaissance du dimère H2A-H2B, qui s'est reconstitué à l'interface des deux protéines déposées sur la membrane de nitrocellulose. L'histone H3 n'est pas reconnue, tandis que un faible halo émane des pistes où les histones H2A et H2B sont à l'état monomérique. Ces résultats montrent que S2, S12, C25, C76 ne reconnaissent pas H3. Ils révèlent en revanche que leur cible est le dimère H2A-H2B, mais pas les monomères.

Le test ci-dessus peut être mis en oeuvre pour sélectionner parmi des VHH obtenus par modifications des séquences SEQ ID N° 1 à 4, ceux présentant la capacité de se lier au dimère H2A-H2B.

EXPERIENCE 4 - Test d'immunofluorescence sur cellules en culture

4.1/ Matériel et méthodes

La lignée HCT116 (adénocarcinome colique humain) et les MEF (fibroblastes embryonnaires murins) sont propagées dans du DMEM (Gibco Life Technologies) supplémenté avec 10 % de sérum de veau foetal et des antibiotiques (pénicilline 10000 unités/ml et streptomycine 10000 $\mu$g/ml de Gibco life Technologies, diluées au 1/100), et incubées dans une atmosphère saturée en $H_2O$ contenant 5 % de $CO_2$.

Pour chaque type cellulaire, les cellules mises en culture sur des lamelles de verre dédiées à l'imagerie cellulaire sont lavées dans du tampon phosphate salin (PBS), puis fixées dans une solution de PBS additionné de paraformaldéhyde à 4 %, pendant 15 min.

Les cellules sont perméabilisées par une incubation de 5 min dans du PBS additionné de 0,25 % Triton@ X100, puis un blocage est effectué dans du PBS 1 % BSA.

Les cellules sont incubées pendant une heure dans un mélange de polypeptide selon l'invention VHH-HA à 5 $\mu$g/ml et d'un anticorps anti-HA (monoclonal souris HA-11 Covance, utilisé à 0,5 $\mu$g/ml). Après trois lavages au PBS, un anticorps secondaire anti-souris couplé à l'Alexa Fluor® 488 (Molecular Probes Life Technologies) dilué au 1/1000 est ajouté sur les lamelles qui sont incubées pendant 45 min à température ambiante.

Les cellules sont lavées 3 fois dans du PBS, le troisième milieu de lavage contenant du DAPI à 1 $\mu$g/ml. Finalement, le montage sur lame est effectué avec du milieu de montage DAKO.

4.2/ Sur cellules humaines

Les polypeptides selon l'invention comprenant les anticorps respectifs S2, S12, C25 et C76 (VHH), fusionnés à l'étiquette HA, et un polypeptide comparatif comprenant le VHH C8, également fusionné à l'étiquette HA, ont été testés sur des cellules humaines HCT116 fixées.

Une observation en microscopie en fluorescence de chacune des lames est réalisée. Les résultats sont montrés sur la figure 6. Ils démontrent clairement que des polypeptides conformes à l'invention, comprenant les anticorps S2, S12, C25 ou C76, génèrent un signal chromatinien sur les cellules humaines fixées, contrairement au polypeptide comparatif C8-HA. L'observation de l'ADN par le signal DAPI est quant à elle identique pour tous les polypeptides. A droite de chaque figure, l'agrandissement d'une cellule en mitose détaille la liaison des polypeptides sur les chromosomes mitotiques.

4.3/ Sur cellules murines

Les polypeptides selon l'invention comprenant les anticorps S2, S12, C25 et C76, fusionnés à l'étiquette HA, ont été testés sur des cellules murines MEF (fibroblastes embryonnaires murins sauvages) fixées, traitées ou non à l'étoposide. Les cellules MEF sont ensemencées sur des lamelles de verre dédiées à l'imagerie. Un jour après l'ensemencement, les cellules sont traitées ou non pendant 1 h à l'étoposide (Sigma Aldrich) dilué dans le milieu de culture à une concentration finale de 10 $\mu$M. Les cellules sont ensuite fixées, perméabilisées, puis incubées avec un mélange VHH-HA et anti-HA, comme précédemment décrit. Afin de contrôler l'efficacité de l'induction de dommages à l'ADN par l'étoposide, une lamelle est immunocolorée avec un anticorps anti-$\gamma$H2AX (Anti phospho histone H2AX Ser[139] monoclonal souris clone JBW301 de Millipore dilué 1/1000 final).

Une observation en microscopie en fluorescence de chacune des lames est réalisée, pour détecter les signaux respectivement générés par le DAPI (montrant l'ADN), l'anti-yH2AX et les polypeptides conformes à l'invention. Les résultats sont montrés sur la figure 7.

Les polypeptides conformes à l'invention (comprenant les anticorps S2, S12, C25 ou C76) génèrent un signal indépendant de la présence de cassures double brin de l'ADN, contrairement au signal obtenu avec l'immunofluorescence anti-$\gamma$H2AX, qui montre les foyers de cassure double brin après traitement à l'étoposide.

EXPERIENCE 5 - Test d'immunofluorescence sur embryons fixés de *Drosophila melanogaster*

Les embryons, obtenus après récolte d'oeufs pondus par des femelles Oregon R sauvages, ont été déchorionés à l'eau de Javel et fixés par du paraformaldéhyde 4 %, puis conservés dans du méthanol à -20 °C. Les embryons sont ensuite réhydratés par 5 lavages en PBS 0,3 % Triton@ X100 et incubés 30 min dans du PAT (PBS 0,3 % Triton@ X100 et 1 % BSA). La solution d'anticorps primaires consiste en un mélange du polypeptide VHH-HA (5 μg/ml final) avec un anticorps anti-HA (monoclonal HA-11 Covance dilué à 0,5 μg/ml) dans le PAT. Ces anticorps primaires sont appliqués toute la nuit sur les embryons à 4 °C. Ces derniers sont ensuite lavés 5 fois 10 min avec du PBS 0,3 % Triton@ X100.

Les anticorps primaires sont révélés par un anticorps anti-souris couplé à l'Alexa 488 (Molecular Probes Life Technologies) dilué au 1/1000 dans du PAT, par incubation pendant 5 h. Le lavage de l'anticorps secondaire est identique à celui des anticorps primaires, excepté que le premier lavage est effectué dans du PBS 0,3 % Triton@ X100 contenant de la RNase A 1 mg/ml, et de l'iodure de propidium 10 μg/ml, afin de marquer l'ADN. Les embryons sont montés dans du milieu de montage DAKO et imagés au microscope confocal Zeiss LSM 510.

Les images obtenues, pour chacun des polypeptides S2-HA, S12-HA, C25-HA et C76-HA conformes à l'invention, et pour le polypeptide comparatif C8-HA, sont montrées sur la figure 8. On y observe que, contrairement au polypeptide comparatif C8-HA, les polypeptides conformes à l'invention permettent un marquage très sélectif de la chromatine.

EXPERIENCE 6 - Test d'immunofluorescence sur le nématode *Caenorhabditis elegans*

Les nématodes *Caenorhabditis elegans* sont collectés et lavés dans du PBS. Les téguments de l'animal sont déstructurés et perméabilisés par la technique de « freeze cracking » entre deux lamelles de verre. Ainsi préparés, les vers sont associés à une lame traitée à la poly L-lysine et fixés par incubation dans un bain d'acétone glacé (5 min) suivi d'un bain de méthanol glacé (5 min). Les vers fixés sont rehydratés dans du PBS avant une incubation d'1 h dans un tampon anticorps PBS 0,5 mM EDTA, 0,5 % Triton@ X100, 1 % BSA, 2 % sérum de veau foetal.

Le mélange d'anticorps primaires utilisé est identique à celui précédemment décrit en référence à l'expérience 5 ci-avant. Les lames sont incubées sur la nuit avec le mélange d'anticorps primaires, puis lavées 3 fois 20 min dans du tampon de lavage (PBS 0,25 % Triton@ X100, 0,1 % BSA).

L'anticorps secondaire anti-souris couplé à l'Alexa 488 dilué au 1/1000 est appliqué sur les lames pendant 5 h. Les lames sont lavées 3 fois 20 min dans du PBS 0,25 % Triton@ X100, 0,1 % BSA. Lors du second lavage, le tampon contient du DAPI (1 μg/ml) pour un marquage de l'ADN.

Le montage final est effectué avec du milieu de montage DAKO, et les nématodes marqués sont imagés au microscope confocal Zeiss LSM 510.

Les images obtenues, pour chacun des polypeptides conformes à l'invention, et pour le polypeptide comparatif C8-HA, sont montrées sur la figure 9. On observe là encore que, contrairement au polypeptide comparatif C8-HA, les polypeptides conformes à l'invention permettent un marquage sélectif de la chromatine.

EXPERIENCE 7 - Test d'immunofluorescence sur la levure *Saccharomyces cerevisiae*

Les levures *Saccharomyces cerevisiae* d'une culture de 50 ml sont fixées par ajout de 7,2 ml de paraformaldéhyde 37 %, et incubation pendant 45 min sous agitation douce. Les cellules sont culottées par centrifugation et lavées 3 fois avec un tampon PBS 1,2 M sorbitol.

Les cellules sont alors remises en suspension dans 1 ml de PBS 1,2 M sorbitol, 28 mM β-mercaptoéthanol, 1 mM fluorure de phénylméthylsulfonyle (PMSF). Les parois sont digérées par l'ajout de 50 μl de zymolyase 10 mg/ml. Après une incubation de 20 min à 37 °C, les cellules sont culottées et lavées avec du PBS 1,2 M sorbitol.

La suspension de sphéroplastes est déposée sur une lamelle de verre recouverte de poly L-lysine, et les cellules ayant adhéré sont fixées par ajout sur la lamelle d'éthanol 70 % refroidi à -20 °C. L'incubation dans l'éthanol est effectuée sur la nuit à -20 °C. Les cellules sont réhydratées par une incubation de la lamelle dans du PBS.

Une perméabilisation des membranes est effectuée par une incubation de 5 min dans du PBS 0,1 % NP40, 0,1 % BSA. Les cellules sont lavées dans du PBS 0,1 % BSA, puis le mélange VHH-HA et anticorps anti-HA dilué dans du PBS 0,1 % BSA est appliqué sur les lamelles. L'incubation de 2 h avec les anticorps primaires est suivie par un lavage dans du PBS.

L'anticorps secondaire anti-souris couplé à l'Alexa 488 dilué au 1/1000 est appliqué sur les lamelles pendant 1 h. Les lamelles sont alors lavées une première fois dans du PBS contenant du DAPI à 1 μg/ml final, puis une seconde fois dans du PBS, avant d'être montées sur une lame à l'aide du milieu de montage DAKO. L'imagerie des levures est effectuée sur un microscope droit champ large (Leica DM5000), à l'objectif 63x.

Les images obtenues, pour chacun des polypeptides S2-HA, S12-HA et C25-HA conformes à l'invention, ainsi que pour le polypeptide comparatif C8-HA, sont montrées sur la figure 10. On observe là encore que, contrairement au polypeptide comparatif C8-HA, les polypeptides conformes à l'invention permettent un marquage sélectif de la chromatine.

EXPERIENCE 8 - Test d'imagerie cellulaire en temps réel sur cellules humaines

L'objectif de cette expérience a été de déterminer si des polypeptides objets de la présente invention, comprenant un VHH fusionné à la protéine fluorescente GFP, pouvaient être exprimés de manière stable sans affecter la capacité des cellules à proliférer, ainsi que d'évaluer les performances de ces VHH-GFP dans le marquage fluorescent de la chromatine de cellules vivantes. Cette expérience a été réalisée avec les VHH conformes à l'invention S2, S12, C25 et C76.

Matériel et méthodes

Construction des vecteurs d'expression

Afin d'exprimer les polypeptides comprenant un domaine VHH en fusion avec la GFP dans les cellules humaines, la séquence codante des VHH est amplifiée par PCR (avec la Phusion® DNA polymérase de New England Biolabs), à l'aide des amorces suivantes :

N1VHH-F :
5' ATACTCGAGCCACCATGGCCCAGGTGCAGCTG 3' (SEQ ID N° 12)

et N1VHH-R :
5' AATGGATCCGCGTAGTCCGGAACGTCGTACG 3' (SEQ ID N° 13)

introduisant les sites XhoI et BamHI en 5' et 3' de la séquence.

Les fragments PCR digérés par ces enzymes sont clonés aux sites XhoI - BamHI du vecteur d'expression pEGFP N1 (Clontech). Afin d'établir par transduction lentivirale des lignées cellulaires exprimant de manière stable les fusions VHH-GFP, les séquences codantes des vecteurs pEGFP N1 ont été sous-clonées dans le vecteur lentiviral navette pTRIP.

Culture cellulaire et transduction lentivirale

Les cellules humaines HT1080 (fibrosarcome) et 293T (lignée de rein embryonnaire transformée) sont propagées dans du DMEM (Gibco Life Technologies) supplémenté avec 10 % de sérum de veau foetal et des antibiotiques (pénicilline et streptomycine, 10 000 U/ml de Life Technologies), et incubées dans une atmosphère saturée en $H_2O$ contenant 5 % de $CO_2$.

Les particules virales sont produites par cotransfection (chlorure de calcium) du vecteur navette pTRIP VHH-GFP avec les vecteurs Gag-Pol 8.91 et VSV-G dans des cellules 293T. Les surnageants cellulaires des cellules tri-transfectées, contenant les particules lentivirales, sont utilisés après titration pour transduire les cellules HT1080.

Imagerie cellulaire en temps réel

Les cellules exprimant un VHH-GFP sont ensemencées (20 000 à 80 000 cellules par puits) dans des chambres de culture dédiées à l'imagerie en temps réel (Labtek 2 puits Nunc), et incubées 24 à 72 h avant imagerie.

La microscopie en temps réel des cellules transgéniques progressant dans le cycle cellulaire est réalisée à l'aide d'un microscope inversé champ large (Zeiss Axio Observer Z1) équipé d'une caméra CoolSnap ES[2], d'une chambre d'incubation à atmosphère contrôlée (en température, hygrométrie, taux de $CO_2$) et d'une platine motorisée pilotée par le logiciel Metamorph. Les cellules sont imagées toutes les 5 min en lumière transmise et en fluorescence par un objectif 20x.

L'imagerie en temps réel de cellules mitotiques est également réalisée à l'aide d'un microscope confocal de type "Laser Scanning" (LSM 510 Zeiss) en position inversée, équipé d'une chambre d'incubation thermostatée identique à celle précédemment décrite, d'une platine motorisée pilotée par le logiciel Zen (Zeiss) et d'un objectif 40x à immersion eau. La fréquence d'acquisition a été de 4 min. Les piles d'images générées sont traitées avec le logiciel Fiji.

Résultats

Les successions d'images ainsi obtenues sont montrées respectivement sur les figures 11A et 11B, pour l'exemple particulier du polypeptide S12-GFP conforme à l'invention. Des résultats similaires ont été obtenus pour les polypeptides S2-GFP, C76-GFP et C25-GFP.

L'expression des transgènes VHH-GFP étant maintenue dans les cellules transduites au fil de leurs passages, ceci montre que la présence des polypeptides objets de l'invention dans la cellule est parfaitement compatible avec le déroulement normal du cycle de division cellulaire.

L'imagerie en temps réel de la fluorescence de la GFP émanant des cellules transgéniques (figure 11A) révèle un marquage spécifique de la chromatine dans les cellules vivantes, semblable à celui obtenu en immunofluorescence dans les cellules fixées. Il est important de noter que la localisation dans le compartiment nucléaire des polypeptides objets de la présente invention ne résulte pas de l'action d'une séquence de localisation nucléaire (NLS), puisque la fusion VHH-GFP n'en possède pas. En revanche, cette phénoménologie est très probablement liée à une diffusion passive à travers les pores de l'enveloppe nucléaire qu'autorise la petite taille de la molécule.

Comme le montre la figure 11A, les fusions VHH-GFP permettent le suivi en imagerie cellulaire, à l'échelle d'une population cellulaire ou de la cellule unique, de la motilité cellulaire, de la forme du noyau, de celle des nucléoles, de la structure de la chromatine.

Plus encore, les polypeptides conformes à l'invention ainsi exprimés dans les cellules permettent le suivi en temps réel de la chorégraphie des chromosomes mitotiques pendant la division cellulaire (figure 11B), autorisant notamment l'étude de défaut de condensation de la chromatine, de ségrégation des chromatides en anaphase, de rotation de la plaque métaphasique, de durée des différentes phases de la mitose. Les polypeptides selon l'invention peuvent ainsi avantageusement être utilisés pour le criblage HCS (High content screening) de banques de composés, ou en RNAi.

Détection des micronoyaux et ponts anaphasiques

Des images plus nettes, après traitement à l'aide du logiciel Image J, obtenues avec le polypeptide S12-GFP, sont montrées sur la figure 15. Sur cette figure, les images successives correspondent à des acquisitions réalisées toutes les 10 min.

On y observe que pendant la mitose, les chromosomes s'alignent sur la plaque métaphasique (images 6-7-8), puis se séparent en deux lots équivalents dans les cellules filles (image 9). Le polypeptide conforme à l'invention permet d'observer la présence de deux micronoyaux (flèche pleine, images 11-18), pendant 1h20, indiquant clairement la présence de lésions cytogénétiques. Une analyse plus fine permet également d'observer un pont anaphasique (flèche pointillée, image 9), caractéristique d'un défaut de mitose.

Ces résultats montrent que le polypeptide conforme à l'invention permet de suivre des défauts de mitose (ponts anaphasiques), mais également des lésions cytogénétiques, de type micronoyaux. Ce polypeptide permet donc la réalisation de tests de génotoxicité nécessitant la visualisation de la chromatine, tel que le test des micronoyaux.

EXPÉRIENCE 9 - Test de mobilité des polypeptides sur leur cible dans des cellules vivantes

L'objet de cette expérimentation a été d'évaluer et de quantifier, par une approche de photo-blanchiment de la GFP effectuée sur les cellules transgéniques VHH-GFP, la mobilité de polypeptides objets de la présente invention S2-GFP, S12-GFP, S45-GFP, C25-GFP et C76-GFP sur leur cible histone dans le noyau des cellules vivantes.

Méthodologie

Les expériences de recouvrement de la fluorescence après photo-blanchiment (FRAP) ont été réalisées sur les cellules HT1080 qui expriment de manière stable les différents VHH-GFP ci-dessus. L'outil optique de photo-blanchiment, d'excitation du fluorophore et d'acquisition de l'émission de photon est un microscope confocal de type "Laser Scanning" (LSM 510 Zeiss) en position inversée, équipé d'une chambre d'incubation thermostatée identique à celle précédemment décrite, et piloté par le logiciel Zen (Zeiss). L'objectif utilisé est un 40x à immersion eau.

Le photo-blanchiment et l'excitation du fluorophore sont réalisés par un Laser Argon accordé à 488 nm. Une sous-région du noyau (Région of Interest : ROI), circulaire, est définie arbitrairement. La séquence FRAP d'illumination - acquisition de la fluorescence consiste en une première acquisition (état initial), puis une illumination par le Laser Argon (488 nm) de la ROI conduisant au photo-blanchiment de la zone est déclenchée, directement suivie d'une acquisition (t = 0) qui se poursuit toutes les secondes jusqu'au retour stabilisé de la fluorescence émise. Pour chaque polypeptide, les mesures de FRAP ont été effectuées sur au moins 10 cellules indépendantes.

Les calculs de la moyenne de l'intensité de la ROI, sa correction, la moyenne relative (moyenne d'intensité / valeur maximale du signal du retour de fluorescence), l'établissement de la courbe de l'intensité relative de la ROI en fonction du temps, puis l'ajustement de cette courbe à une relation mathématique ($^\#$) reliant l'intensité en fonction du temps (permettant de déduire la valeur du temps de demi-recouvrement $\tau_{1/2}$) sont effectués par la macro FRAP du logiciel Zen. Le calcul des moyennes et écarts-types (de 10 expériences indépendantes pour chaque polypeptide) des valeurs d'intensité relative en fonction du temps et des $\tau_{1/2}$ déduits, l'établissement des courbes et de l'histogramme afférents ont été réalisés à l'aide du tableur Excel.

$$(^\#) \; I(t) = I_E - I_1 \times \exp(-t/\tau)$$

où $I_E$ est la valeur au plateau du retour, $I_1$ est la valeur de la fraction mobile, $\tau$ est la constante de temps qui décrit la mobilité de la molécule étudiée, $\tau_{1/2}$ est le temps de demi-retour de la fluorescence de la molécule fluorescente photo-blanchie avec $\tau_{1/2} = \ln 0,5/-\tau$.

Résultats

Les courbes du recouvrement de la fluorescence en fonction du temps, pour chaque polypeptide conforme à l'invention, sont montrées sur la figure 12.

Les valeurs du temps déduit de demi-retour $\tau_{1/2}$ du polypeptide sur sa cible, pour chacun des polypeptides testés, sont présentées sur la figure 13.

L'échelle de valeur des $\tau_{1/2}$, entre 3 et 1,5 secondes, indique une mobilité relativement importante des polypeptides conformes à l'invention sur leur cible histone.

EXPÉRIENCE 10 - Test de spécificité vis-à-vis des dimères majeurs de H2A-H2B

La spécificité de liaison des polypeptides conformes à l'invention S2 et S12 aux dimères H2AX-H2B et H2AZ-H2B a été étudiée par une approche de type ELISA.

Les histones humaines H2A, H2AX, H2AZ, et H2B ont été obtenues par une co-expression du dimère H2A-H2B dans la bactérie *E. coli* suivie de deux étapes de purification par chromatographie d'affinité, selon une procédure décrite dans la publication de Anderson et al. (2010).

Les VHH étiquetés HA et $His_6$ ont été produits par sécrétion chez la bactérie *E. coli*. Le polypeptide a été purifié du milieu bactérien par une précipitation au sulfate d'ammonium suivie par une chromatographie d'affinité sur une résine de cobalt.

Pour le test ELISA, les trois types de dimères H2A-H2B, H2AX-H2B et H2AZ-H2B, ont été dilués dans du PBS à 1 $\mu$g/100$\mu$l, puis 100 $\mu$l de chaque dilution de dimère ont été distribués en duplicat dans les puits d'une plaque 96 puits de type Maxisorp® (Nunc). L'adsorption des dimères d'histones au plastique a été effectuée sur la nuit à 4 °C. Le blocage des puits a été effectué à l'aide de 100 $\mu$l de PBS + 10% de lait écrémé pendant 2 h. Les puits ont alors été lavés à l'aide de 100 PBS + 0,1% Tween® 20 (PBST). 100 $\mu$l de PBST + 5% de lait écrémé contenant le polypeptide VHH-étiqueté HA purifié dilué à 1 ng/ml et un anticorps anti-HA dilué à 0,5 ng/ml ont été ajoutés dans les puits.

Un anticorps anti-H2B (anticorps monoclonal de lapin (D2H6) Cell Signaling #12364) a été utilisé comme contrôle à la fois positif, et de charge des différents puits.

Après une incubation à température ambiante pendant 1 h, les puits ont été lavés avec 100 $\mu$l de TBST, et 100 $\mu$l d'anticorps secondaire anti-souris couplé à la HRP dilué au 1/5000 dans du TBST ont été ajoutés aux puits. La plaque a été incubée 45 min, puis deux lavages à l'aide de 100 $\mu$l/puits de TBST ont été effectués. L'ajout de 100 $\mu$l du substrat TMB (Sigma Aldrich) a permis la révélation de l'activité HRP associée à l'anticorps secondaire. L'intensité du signal dans chaque puits a été quantifiée par la lecture de la $DO_{450nm}$ dans un lecteur de plaque (Multiskan®). L'analyse des valeurs de densité optique (DO), et en particulier le calcul des moyennes, a été effectuée à l'aide du tableur Excel.

Les résultats obtenus sont montrés sur la figure 14.

Les graphiques A et B montrent que les polypeptides S2 et S12 lient de manière non significativement différente les trois types de dimère H2A-H2B. Les intensités de signal similaires entre les puits des trois dimères d'histones obtenues avec le contrôle anti-H2B commercial (graphique C) montrent par ailleurs que les trois types de dimères d'histones sont présents en quantité équivalente dans les puits du dispositif expérimental.

EXPÉRIENCE 11 - Imagerie de la chromatine d'un organisme drosophile transgénique modèle

Une drosophile transgénique exprimant de manière ubiquitaire le polypeptide conforme à l'invention S12-HA-GFP a été générée.

Matériel et méthode

Clonage du vecteur d'expression

Afin de pouvoir procéder à l'insertion site spécifique dans le génome de la mouche *Drosophila melanogaster* d'une cassette permettant l'expression du S12-GFP sous le système d'expression UAS - Gal4 (Phelps et al. (1998)), la séquence codante de la fusion S12-HA-GFP obtenue à partir de la construction pEGFPN1 S12-HA (décrit dans l'Expérience 8) a été clonée en utilisant les sites de restriction Xhol - Xbal dans le vecteur de transgénèse dédié pUAS (Bischof et al. (2007)).

Génération des mouches transgéniques et expression du transgène

L'insertion du transgène S12-HA-GFP locus 68A4 du chromosome 3 a été réalisée par la microinjection du plasmide pUAS S12-HA-GFP dans les cellules germinales d'embryons (nosC31 NLS;attP2) qui expriment l'intégrase phiC31 (Bischof et al. (2007)). L'expression ubiquitaire du transgène a été réalisée par un driver Actine ou Ubiquitine.

Imagerie live en fluorescence de la drosophile

Pour l'imagerie de l'embryon vivant, les oeufs ont été déchorionés manuellement, avant d'être positionnés dans une goutte d'huile (Halocarbon) sur une lame de microscopie. L'imagerie de la fluorescence a été effectuée à l'aide d'un objectif 25X d'un microscope confocal (LSM 510 Zeiss) équipé une enceinte thermorégulée à 25 °C. Pour l'imagerie en temps réel du développement embryonnaire, les acquisitions ont été effectuées toutes les 2 min. L'imagerie des larves, et des mouches adultes vivantes exprimant S12-HA-GFP a été effectuée à l'aide d'un microscope Macrofluo® (Leica) équipé pour une illumination de l'échantillon en épifluorescence, et pourvu d'une caméra CoolSNAP® ES2. Les images générées ont été traitées à l'aide du logiciel Image J.

Résultats

L'imagerie confocale de la fluorescence émise par des embryons vivants exprimant le polypeptide S12-HA-GFP a été effectuée. Un marquage fluorescent spécifique de la chromatine est observé.

La figure 16 montre des images de microscopie en temps réel prises toutes les 2 min d'un embryon au stade blastoderme syncytial qui effectue un cycle de division cellulaire (synchrones à ce stade du développement). On y observe que le polypeptide sonde S12-HA-GFP permet de visualiser les mitoses synchrones à ce moment du développement embryonnaire. L'image au temps '0 min' montre comment la sonde S12-HA-GFP permet la visualisation des noyaux interphasiques. Dans l'image au temps '4 min', les plaques métaphasiques sont reconnaissables, et dans l'image au temps '6 min', la sonde permet de visualiser clairement des figures d'anaphase (indiquées par une flèche dans la partie droite de l'embryon). Dans les images acquises plus tard (temps '10 min'), le doublement du nombre de noyaux est clairement observable.

La figure 17 montre cinq images prises à 2 h d'intervalle les unes des autres provenant d'une expérience de microscopie en temps réel d'une durée totale de 15 h, avec une fréquence d'acquisition de 2 min, d'un embryon exprimant la sonde S12-HA-GFP. Ces images montrent que la fusion VHH-GFP reste spécifiquement associée à la chromatine pendant tout le développement embryonnaire, et démontrent les performances de l'outil dans la visualisation sur des temps longs de la chromatine.

Les larves issues de ces embryons fluorescents ont été observées par microscopie en épi-fluorescence. L'image obtenue est montrée sur la figure 18. La fluorescence se trouve spécifiquement concentrée au niveau des noyaux qui peuvent être observés par transparence. En particulier, les noyaux géants des glandes salivaires sont clairement visibles, et indiqués par une flèche sur la figure.

La figure 19 montre une image acquise par_microscopie en épifluorescence d'un adulte issu de la métamorphose d'une larve exprimant la sonde S12-HA-GFP. Cet adulte est parfaitement vivant et il continue d'exprimer le transgène.

Ces résultats d'imagerie effectuée pendant le développement d'une drosophile exprimant de manière ubiquitaire le polypeptide conforme à l'invention S12-GFP démontrent que ce polypeptide marque de manière spécifique la chromatine chez un invertébré et ce de manière similaire à ce qui a pu être imagé dans les cellules humaines. En outre, le polypeptide reste associé à la chromatine pendant tout le développement jusqu'au stade adulte. L'expression du polypeptide n'est pas invasive, c'est-à-dire qu'elle n'empêche pas le développement de la drosophile du stade embryonnaire jusqu'au stade adulte. Ces résultats montrent clairement que le polypeptide conforme à l'invention peut être utilisé pour une visualisation de la chromatine avec une haute résolution tant spatiale que temporelle dans des processus cellulaires extrêmement dynamiques.

EXPÉRIENCE 12 - « Diabodies »

Des polypeptides conformes à l'invention utiles pour l'imagerie de la chromatine ont été générés par concaténation de VHH autour de la GFP.

Matériel et méthode

Clonage des constructions « diabodies » fluorescents

Afin de placer un domaine VHH de part et d'autre de la GFP, la stratégie a été de cloner aux sites Nhel - BsrG1 du vecteur pEGFPC1 VHH le fragment Nhel - BsrG1 provenant du vecteur pEGFPN1 VHH. Les nouveaux vecteurs d'expression ainsi générés concernent les VHH suivants : S2, S12, S45, et C25. Ils ont été nommés respectivement pEGFP S2-GFP-S2, pEGFP S12-GFP-S12, pEGFP S45-GFP-S45, pEGFP C25-GFP-C25.

Expression cellulaire et étude de la dynamique des sondes par analyse de la vitesse de retour de fluorescence après photo-blanchiment (FRAP : Fluorescence Recovery After Photobleaching).

Afin d'exprimer dans des cellules humaines les fusions diabodies et de procéder à leur imagerie en temps réel, des cellules HCT116 ont été transfectées par les vecteurs pEGFP VHH-GFP-VHH (en utilisant l'agent de transfection JetPEI®) en format Lab-Tek® 2 puits (Nunc). L'imagerie des cellules transfectées vivantes, et les expériences de FRAP ont été menées avec un objectif 40x à immersion d'un microscope confocal (LSM 510 Zeiss). La séquence des opérations pour le FRAP a été la suivante :

1) acquisition pré photo-blanchiment,

2) illumination d'une région définie du noyau en forme de cercle conduisant à une extinction de l'émission de fluorescence dans la zone,

3) la séquence de photo-blanchiment a été immédiatement suivie par une séquence d'acquisition d'une fréquence de 1 s pendant 1 min.

L'établissement des valeurs d'intensité corrigées à partir des données brutes d'intensité moyenne dans la région photo-blanchie a été effectué à l'aide du module FRAP du logiciel Zen (Zeiss). Ce logiciel corrige les données brutes d'intensité pour tenir compte du photo-blanchiment lié à la séquence d'acquisition, et du bruit de fond. Le calcul du temps de demi-retour de fluorescence ($T_{1/2}$) a été effectué par le logiciel par une adéquation à une fonction mathématique mono-exponentielle :

$$I(t) = I_E - I_1 \times \exp(-t/\tau)$$

dans laquelle $I_E$ est la valeur au plateau du retour, $I_1$ est la valeur de la fraction mobile, $\tau$ est la constante de temps qui décrit la mobilité de la molécule étudiée, $\tau_{1/2}$ est le temps de demi-retour de la fluorescence de la molécule fluorescente avec $\tau_{1/2} = \ln 0,5/-\tau$.

Les graphiques ont été établis à l'aide du tableur Excel. Les images ont été traitées par Image J.

Résultats

La figure 20 présente des montages d'images d'expériences de FRAP réalisées sur des cellules HCT116 exprimant les fusions VHH-GFP-VHH, mais aussi la construction S12-GFP comme contrôle représentatif d'une sonde qui ne possède qu'un module VHH. Dans ces conditions de vitesse d'acquisition des images, on remarque que l'extinction en forme de cercle dans le noyau résultant du photo-blanchiment est bien visible avec les sondes « diabodies », ce qui est symptomatique d'une mobilité plus réduite. En revanche, dans les mêmes conditions expérimentales, ce photo-blanchiment apparaît diffus avec la sonde S12-GFP, un phénomène lié à une importante dynamique. De plus, visuellement, le recouvrement de la fluorescence est plus lent dans les sondes « diabodies » comparé à S12-GFP. Enfin, l'examen visuel des montages permet également de voir des différences entre les 4 constructions VHH-GFP-VHH dans la vitesse de recouvrement.

La quantification des intensités a permis d'établir des courbes de cinétiques de retour de la fluorescence, pour une moyenne effectuée sur 9 cellules indépendantes pour chaque sonde. Ces quantifications montrent clairement que le recouvrement de la fluorescence est plus lent avec les sondes « diabodies », en comparaison avec la sonde mono-VHH S12-GFP contrôle. Le temps de demi-recouvrement ($T_{1/2}$) a été calculé à partir de chaque courbe de retour de la fluorescence. La moyenne des valeurs établis pour chaque construction est présentée dans la figure 21. Le graphique y montre que d'une manière générale, les sondes « diabodies » ont un temps de demi-recouvrement significativement plus long que la sonde mono-VHH S12-GFP. En particulier, le $T_{1/2}$ de la version « diabody » de S12 est quatre fois plus important que celui de S12-GFP. Le graphique montre également une importante disparité entre les sondes « diabodies », la sonde S2-GFP-S2 étant la moins mobile, tandis que S45-GFP-S45 est la plus mobile des constructions « diabodies ».

La plupart des « diabodies », et en particulier ceux comprenant S2 et S12, permettent en outre de visualiser des compartiments nucléaires que ne révèle pas les polypeptides mono-VHH, comme on peut l'observer sur la figure 20. La figure 22 montre une image représentative de ce que permet de visualiser la sonde S12-GFP-S12 dans le noyau d'une cellule humaine. Ce patron de signal - un marquage péri-nucléolaire et péri-nucléaire - est symptomatique d'une sonde qui reconnaît préférentiellement l'hétérochromatine.

Ces résultats démontrent que les polypeptides selon l'invention associant deux modules VHH sur la même chaine peptidique présentent une mobilité moindre sur la chromatine que les polypeptides comprenant un seul module VHH. Ces polypeptides constituent des outils uniques pour visualiser en temps réel, dans les cellules vivantes, la dynamique des domaines d'hétérochromatine.

EXPÉRIENCE 13 - Modification de l'état de la chromatine - test d'immunofluorescence sur cellules en culture

L'objectif de cette expérience est de démontrer que le couplage d'une activité aux polypeptides objets de la présente invention permet de modifier l'état global de la chromatine.

A cet effet, il est généré un polypeptide dans lequel S12 est couplé à la protéine RNF8, une E3 ubiquitine ligase spécifiquement recrutée aux sites de cassures double-brins de l'ADN (CDBs), où elle est essentielle pour l'ubiquitinylation des histones H2A et H2AX. Les histones H2A et H2AX localement ubiquitinylées permettent le recrutement aux sites de CDBs des protéines 53BP1 et BRCA1, deux facteurs clés de la réparation des CDBs (Mailand et al. (2007)).

La construction S12-RNF8, ainsi que les protéines S12 et RNF8 seules, ont été exprimées en cellule humaine de façon transitoire afin de vérifier si le recrutement de 53BP1 et BRCA1 était altéré lorsque le VHH S12 est fusionné à RNF8.

Matériel et méthodes

Construction des vecteurs d'expression

La séquence codante du VHH S12 a été sous-clonée dans un vecteur d'expression eucaryote en fusion avec la protéine fluorescente mCherry en C-terminal, pour former le plasmide appelé pAO-S12-mCherry.

La séquence codante de RNF8 préalablement insérée dans le vecteur pEGFP-C1 (Mailand et al. (2007)) a été sous-clonée dans le vecteur pmCherry-C1 pour former le plasmide appelé pmCherry-RNF8.

La séquence codante de mCherry-RNF8 provenant du plasmide pmCherry-RNF8 a été sous-clonée dans le vecteur pEGFP-N1 contenant la séquence codante du VHH S12 (décrit dans l'Expérience 8), en lieu et place de la séquence codante de la EGFP, pour former le plasmide appelé pS12-mCherry-RNF8.

Culture et imagerie cellulaire

La lignée HT1080 (fibrosarcome humain) a été propagée dans du milieu DMEM (Gibco Life Technologies) supplémenté avec 10 % de sérum de veau foetal et des antibiotiques (pénicilline 10000 unités/ml et streptomycine 10000 $\mu$g/ml de Gibco life Technologies, diluées au 1/100), et incubées dans une atmosphère saturée en $H_2O$ contenant 5 % de $CO_2$.

Les différents plasmides ont été transfectés au moyen du réactif TransIT®-2020 (Mirus) dans les cellules mises en culture sur des lamelles de verre dédiées à l'imagerie cellulaire, afin de permettre l'expression transitoire des constructions pendant 24 à 42 h. Selon l'expérience, les cellules ont été traitées pendant 1 h avec 10 picoM de calichéamicine (Wyeth), pour induire la formation de CDBs, avant d'être lavées dans du tampon phosphate salin (PBS), puis fixées dans une solution de PBS additionné de paraformaldéhyde à 4 %, pendant 20 min.

Les cellules ont été perméabilisées par une incubation de 15 min dans du PBS additionné de 0, 5 % Triton® X100, puis un blocage a été effectué dans du PBS 10 % goat sérum.

Les cellules ont été incubées pendant 1 h avec un anticorps soit anti-53BP1 (polyclonal lapin NB100-304 Novus Biologicals, utilisé à 0,33 pg/ml), soit anti-BRCA1 (monoclonal souris sc-6954 Santa Cruz Biotechnology, utilisé à 2 μg/ml), soit anti-γH2AX (monoclonal souris JBW301 Merck/Millipore, utilisé à 0,33 μg/ml). Après trois lavages au PBS, un anticorps secondaire anti-souris ou anti-lapin couplé à l'Alexa Fluor® 488 (Molecular Probes Life Technologies) dilué au 1/1000 a été ajouté sur les lamelles qui sont incubées pendant 45 min à température ambiante.

Les cellules ont été lavées 3 fois dans du PBS, le troisième milieu de lavage contenant du DAPI à 1 μg/ml. Finalement, le montage sur lame a été effectué avec du milieu de montage DAKO. L'imagerie des cellules en fluorescence a été réalisée comme décrit précédemment.

Résultats

La figure 23 montre les images obtenues, pour une détection respectivement par les anticorps anti-γH2AX, anti-53BP1 et par l'agent intercalant DAPI, pour des cellules respectivement non traitées et traitées par la calichéamicine.

On y observe que les CDBs peuvent être visualisées en cellule grâce à l'observation de différents biomarqueurs tels que γH2AX (forme phosphorylée sur la sérine 139 du variant d'histone H2AX) et 53BP1. En absence de traitement, les cellules ne subissent pas de CDB et ne présentent aucun signal γH2AX, alors que 53BP1 est retrouvé de façon diffuse dans l'ensemble du noyau (figure 23 en haut). Le traitement à la calichéamicine, en induisant des CDBs, conduit à une phosphorylation de H2AX dans la totalité des cellules, se traduisant par un marquage γH2AX majoritairement ponctiforme (sous forme de foyers), qui marque les sites de CDBs (figure 23 en bas). 53BP1 forme également des foyers qui se colocalisent avec les foyers γH2AX. Dans certaines cellules, ce marquage n'apparaît pas sous forme de foyers (flèche blanche), dû à un trop grand nombre de CDBs ou à un défaut de recrutement de 53BP1.

La proportion de cellules présentant des foyers 53BP1 en réponse à la calichéamicine a été calculée, pour les cellules exprimant respectivement les polypeptides S12, RNF8 et S12-RNF8. Les résultats obtenus sont montrés sur la figure 24. On y observe que lorsque les cellules expriment le VHH S12, la proportion de cellules présentant des foyers 53BP1 en réponse à la calichéamicine est proche de 70 %. Cependant, cette proportion baisse à 28 % lorsque les cellules expriment RNF8, et à 10 % lorsque les cellules expriment S12-RNF8.

La proportion de cellules présentant des foyers BRCA1, en phase S de réplication de l'ADN, a également été calculée, pour les cellules exprimant respectivement les polypeptides S12, RNF8 et S12-RNF8. Les résultats obtenus sont montrés sur la figure 25. On y observe que 27 % des cellules exprimant S12 présentent des foyers BRCA1, ce qui représente la proportion attendue de cellules en phase S pour des cellules en culture. En revanche, seules 9 % et 1 % de cellules exprimant respectivement RNF8 et S12-RNF8 montrent des foyers BRCA1.

Afin de vérifier que les cellules exprimant la construction S12-RNF8 présentent un défaut de réparation des CDBs, le signal γH2AX a été analysé plus en détail. La figure 26 montre la proportion de cellules montrant un signal γH2AX, pour les cellules exprimant respectivement les polypeptides S12, RNF8 et S12-RNF8, pour des durées d'expression de 24 h et 42 h. Il apparaît que même en absence de dommages exogènes, une forte proportion de cellules exprimant S12-RNF8 présente un signal γH2AX comparé aux cellules exprimant S12 ou RNF8. De plus, cette proportion de cellules positives pour γH2AX augmente avec la durée d'expression de la construction. Ce résultat indique que les cellules qui expriment S12-RNF8 accumulent au cours du temps des CDBs non réparées.

Les résultats ci-avant montrent que les polypeptides conformes à l'invention comprenant le VHH S12 permettent de modifier la fonction d'une protéine en modifiant sa localisation. Dans le cas présent, RNF8 fusionné à S12 ne peut plus être localisé spécifiquement aux sites de CDB, car le VHH le dirige continuellement sur l'ensemble de la chromatine. De ce fait, la fonction ubiquitine ligase de RNF8 est diluée à l'ensemble de la chromatine, et ne peut donc plus être enrichie aux sites de CDB. En résulte une incapacité pour les protéines 53BP1 et BRCA1 d'être normalement recrutées aux CDBs, et donc un défaut de réparation de ces lésions, ce qui explique l'accumulation du signal γH2AX. Les polypeptides selon l'invention peuvent donc être utilisés pour altérer l'état général de la chromatine, en détournant le recrutement spécifique de protéines de modification de la chromatine.

**REFERENCES BIBLIOGRAPHIQUES**

Anderson et al. (2010) Protein expression and purification, 72(2): 194-204

Arbabi Ghahroudi et al. (1997) FEBS Lett., 414(3): 521-6

Bischof et al. (2007) Proc. Natl. Acad. Sci. U. S. A. 104: 3312-3317

Chiu et al. (2010) Molecular Pharmacology, 77(4): 497-507

Kirsch-Volders (1997) Mutation Res., 392: 1-4

Lee et al. (2007) Nature Protocols, 2(11): 3001-8

Mailand et al. (2007) Cell, 131(5): 887-900

Phelps et al. (1998) Methods, 14: 367-379

Shechter et al. (2007) Nature Protocols, 2(6): 1445-57

Sirven et al. (2001) Mol Ther., 3(4): 438-48

## Revendications

1. Polypeptide comprenant un anticorps à domaine unique dirigé contre la chromatine, comprenant le domaine variable d'un anticorps à chaîne lourde naturellement dépourvu de chaîne légère (VHH) de camélidé capable de se lier spécifiquement à un complexe d'histones H2A et H2B.

2. Polypeptide selon la revendication 1, dans lequel ledit anticorps à domaine unique présente :

    - la séquence d'acides aminés de l'une quelconque des séquences SEQ ID N° 1, SEQ ID N° 2, SEQ ID N° 3 ou SEQ ID N° 4,
    - une séquence comprenant une ou plusieurs délétion(s), addition(s) ou substitution(s) d'un ou plusieurs acides aminés par rapport à l'une de ces séquences, ledit anticorps présentant dans un test d'immunoadsorption par enzyme liée (ELISA) dirigé contre un complexe d'histones H2A et H2B, une performance d'au moins 50 % par rapport à l'un ou l'autre des anticorps de séquence SEQ ID N° 1 ou SEQ ID N° 2,
    - et/ou une partie fonctionnelle d'une desdites séquences conservant le(s) site(s) de liaison et le(s) domaine(s) protéique(s) nécessaires à la liaison à un complexe d'histones H2A et H2B, présentant dans un test d'immunoadsorption par enzyme liée (ELISA) dirigé contre un complexe d'histones H2A et H2B, une performance d'au moins 50 % par rapport à l'un ou l'autre des anticorps de séquence SEQ ID N° 1 ou SEQ ID N° 2.

3. Polypeptide selon l'une quelconque des revendications 1 à 2, dans lequel ledit anticorps à domaine unique est un domaine VHH.

4. Polypeptide selon la revendication 3, dans lequel ledit anticorps à domaine unique comporte, en position 107 selon la numérotation de Kabat, un résidu arginine.

5. Polypeptide selon l'une quelconque des revendications 3 à 4, dans lequel ledit anticorps à domaine unique comporte, en position 110 selon la numérotation de Kabat, un résidu sérine ou un résidu thréonine.

6. Polypeptide selon l'une quelconque des revendications 3 à 5, dans lequel ledit anticorps à domaine unique comporte, en position 104 selon la numérotation de Kabat, un résidu glycine.

7. Polypeptide selon l'une quelconque des revendications 3 à 6, dans lequel ledit anticorps à domaine unique comporte, en position 105 selon la numérotation de Kabat, un résidu sérine ou un résidu tyrosine.

8. Polypeptide selon l'une quelconque des revendications 1 à 7, comprenant une pluralité d'anticorps à domaine unique dirigés contre la chromatine, chacun desdits anticorps comprenant le domaine variable d'un anticorps à chaîne lourde naturellement dépourvu de chaîne légère (VHH) de camélidé capable de se lier spécifiquement à un complexe d'histones H2A et H2B.

9. Polypeptide selon l'une quelconque des revendications 1 à 8, comprenant en outre une séquence peptidique correspondant à un peptide / protéine fonctionnel d'intérêt.

10. Polypeptide selon l'une quelconque des revendications 1 à 9, comprenant en outre une séquence peptidique correspondant à un peptide de pénétration cellulaire.

11. Molécule d'acide nucléique codant pour un polypeptide selon l'une quelconque des revendications 1 à 10.

12. Utilisation d'un polypeptide selon l'une quelconque des revendications 1 à 10, et/ou d'une molécule d'acide nucléique selon la revendication 11, pour la détection et/ou la visualisation en temps réel de la chromatine.

13. Utilisation selon la revendication 12, d'un polypeptide selon les revendications 9 et 10, dans lequel ladite protéine fonctionnelle d'intérêt est une protéine détectable par fluorescence, luminescence ou phosphorescence, pour la visualisation en temps réel de la chromatine dans des cellules vivantes.

14. Utilisation d'un polypeptide selon l'une quelconque des revendications 1 à 10, et/ou d'une molécule d'acide nucléique selon la revendication 11, pour la visualisation des perturbations du profil mitotique de la chromatine.

15. Kit, notamment pour la détection et/ou visualisation de la chromatine, comprenant un polypeptide selon l'une quelconque des revendications 1 à 10, et/ou une molécule d'acide nucléique selon la revendication 11.

**Patentansprüche**

1. Polypeptid, umfassend einen gegen Chromatin gerichteten Einzeldomänenantikörper, der die variable Domäne eines von Natur aus leichtkettenfreien Schwerketten-Antikörpers (VHH) von Kameliden umfasst, der in der Lage ist, sich spezifisch an einen Komplex der Histone H2A und H2B zu binden.

2. Polypeptid nach Anspruch 1, wobei der Einzeldomänenantikörper Folgendes aufweist:

    - die Aminosäuresequenz einer der Sequenzen SEQ ID NO: 1, SEQ ID NO: 2, SEQ ID NO: 3 oder SEQ ID NO: 4,
    - eine Sequenz, die eine oder mehrere Deletion(en), Addition(en) oder Substitution(en) einer oder mehrerer Aminosäuren in Bezug auf eine dieser Sequenzen umfasst, wobei der Antikörper in einem enzymgebundenen Immunoadsorptionstest (ELISA), der gegen einen Komplex der Histone H2A und H2B gerichtet ist, eine Leistung von mindestens 50 % im Vergleich zu einem der beiden Antikörper der Sequenz SEQ ID NO: 1 oder SEQ ID NO: 2 aufweist,
    - und/oder einen funktionellen Teil einer der Sequenzen, der die Bindungsstelle(n) und Proteindomäne(n), die für die Bindung an einen Komplex der Histone H2A und H2B erforderlich sind, beibehält und in einem enzymgebundenen Immunoadsorptionstest (ELISA) gegen einen Komplex der Histone H2A und H2B eine Leistung von mindestens 50 % im Vergleich zu einem der Antikörper mit der Sequenz SEQ ID NO: 1 oder SEQ ID NO: 2 aufweist.

3. Polypeptid nach einem der Ansprüche 1 bis 2, wobei es sich bei dem Einzeldomänenantikörper um eine VHH-Domäne handelt.

4. Polypeptid nach Anspruch 3, wobei der Einzeldomänenantikörper an Position 107 gemäß der Kabat-Nummerierung einen Argininrest aufweist.

5. Polypeptid nach einem der Ansprüche 3 bis 4, wobei der Einzeldomänenantikörper an Position 110 gemäß der Kabat-Nummerierung einen Serinrest oder einen Threoninrest aufweist.

6. Polypeptid nach einem der Ansprüche 3 bis 5, wobei der Einzeldomänenantikörper an Position 104 gemäß der Kabat-Nummerierung einen Glycinrest aufweist.

7. Polypeptid nach einem der Ansprüche 3 bis 6, wobei der Einzeldomänenantikörper an Position 105 gemäß der Kabat-Nummerierung einen Serinrest oder einen Tyrosinrest aufweist.

8. Polypeptid nach einem der Ansprüche 1 bis 7, umfassend eine Vielzahl von gegen Chromatin gerichteten Einzeldomänenantikörpern, wobei jeder der Antikörper die variable Domäne eines von Natur aus leichtkettenfreien Schwerkettenantikörpers (VHH) von Kameliden umfasst, der in der Lage ist, sich spezifisch an einen Komplex der Histone H2A und H2B zu binden.

9. Polypeptid nach einem der Ansprüche 1 bis 8, das außerdem eine Peptidsequenz umfasst, die einem funktionellen Peptid/Protein von Interesse entspricht.

10. Polypeptid nach einem der Ansprüche 1 bis 9, das zusätzlich eine Peptidsequenz umfasst, die einem zellpenetrierenden Peptid entspricht.

11. Nukleinsäuremolekül, das für ein Polypeptid nach einem der Ansprüche 1 bis 10 codiert.

12. Verwendung eines Polypeptids nach einem der Ansprüche 1 bis 10 und/oder eines Nukleinsäuremoleküls nach Anspruch 11 zum Nachweis und/oder zur Echtzeitvisualisierung von Chromatin.

13. Verwendung eines Polypeptids nach Anspruch 12 gemäß den Ansprüchen 9 und 10, wobei das funktionelle Protein von Interesse ein Protein ist, das zur Echtzeitvisualisierung von Chromatin in lebenden Zellen durch Fluoreszenz, Lumineszenz oder Phosphoreszenz nachweisbar ist.

14. Verwendung eines Polypeptids nach einem der Ansprüche 1 bis 10 und/oder eines Nukleinsäuremoleküls nach Anspruch 11 für die Visualisierung von Störungen des mitotischen Profils von Chromatin.

15. Kit, insbesondere zum Nachweis und/oder zur Visualisierung von Chromatin, umfassend ein Polypeptid nach einem der Ansprüche 1 bis 10 und/oder ein Nukleinsäuremolekül nach Anspruch 11.

**Claims**

1. A polypeptide comprising a single-domain antibody directed against chromatin, comprising the variable domain of a heavy-chain antibody naturally devoid of a light chain (VHH) of a camelid capable of binding specifically to a complex of H2A and H2B histones.

2. A polypeptide as claimed in claim 1, wherein said single-domain antibody has:

- the amino acid sequence of any one of the sequences SEQ ID No. 1, SEQ ID No. 2, SEQ ID No. 3 or SEQ ID No. 4,
- a sequence comprising one or more deletion(s), addition(s) or substitution(s) of one or more amino acids with respect to one of these sequences, said antibody exhibiting in an enzyme-linked immunoabsorbent assay (ELISA) against a complex of H2A and H2B histones, a performance level of at least 50% compared with either one of the antibodies of sequences SEQ ID No. 1 or SEQ No. 2,
- and/or a functional portion of one of said sequences which retains the binding site(s) and the protein domain(s) required for binding to a complex of H2A and H2B histones, exhibiting in an enzyme-linked immunoabsorbent assay (ELISA) against a complex of H2A and H2B histones, a performance level of at least 50% compared with either one of the antibodies of sequences SEQ ID No. 1 or SEQ No. 2.

3. A polypeptide as claimed in any one of claims 1 and 2, wherein said single-domain antibody is a VHH domain.

4. A polypeptide as claimed in claim 3, wherein said single-domain antibody comprises, in position 107 according to Kabat numbering, an arginine residue.

5. A polypeptide as claimed in any one of claims 3 and 4, wherein said single-domain antibody comprises, in position 110 according to Kabat numbering, a serine residue or a threonine residue.

6. A polypeptide as claimed in any one of claims 3 to 5, wherein said single-domain antibody comprises, in position 104 according to Kabat numbering, a glycine residue.

7. A polypeptide as claimed in any one of claims 3 to 6, wherein said single-domain antibody comprises, in position 105 according to Kabat numbering, a serine residue or a tyrosine residue.

8. A polypeptide as claimed in any one of claims 1 to 7, comprising a plurality or single-domain antibodies directed against chromatin, each of said antibodies comprising the variable domain of a heavy-chain antibody naturally devoid of a light chain (VHH) of a camelid capable of binding specifically to a complex of H2A and H2B histones.

9. A polypeptide as claimed in any one of claims 1 to 8, also comprising a peptide sequence corresponding to a functional peptide/protein of interest.

10. A polypeptide as claimed in any one of claims 1 to 9, also comprising a peptide sequence corresponding to a cell-penetrating peptide.

11. A nucleic acid molecule encoding a polypeptide as claimed in any one of claims 1 to 10.

12. The use of a polypeptide as claimed in any one of claims 1 to 10, and/or of a nucleic acid molecule as claimed in claim 11, for detecting and/or visualizing chromatin in real time.

13. The use as claimed in claim 12, of a polypeptide as claimed in claims 9 and 10, wherein said functional protein of interest is a protein detectable by fluorescence, luminescence or phosphorescence, for visualizing chromatin in real time in living cells.

14. The use of a polypeptide as claimed in any one of claims 1 to 10, and/or of a nucleic acid molecule as claimed in claim 11, for visualizing chromatin mitotic profile disruptions.

15. A kit, in particular for detecting and/or visualizing chromatin, comprising a polypeptide as claimed in any one of claims 1 to 10, and/or a nucleic acid molecule as claimed in claim 11.

FIG 1

|  | FR1 | CDR1 | FR2 | CDR2 | |
|---|---|---|---|---|---|
| C25 | MAQVQLQASGGGLVQAGESLTLSCDASG | RTFSIGA | MGWFRQAPGKEREFLAAI | NWNGGYTS | 61 |
| C76 | MAQVQLQASGGGLVQAGGSLRLSCAVSG | GTLSSYT | MGWFRQAPGKEREFVSAL | SRVGGKSD | 61 |
| S2 | MAQVQLQASGGGLVQAGDSLRLSCAAAG | RSFSSYA | MGWFRQAPGKEREFVAAI | SRSRGVTY | 61 |
| S12 | MAQVQLQASGGGLVQAGGSLRLSCAASG | RTVAT | LGWFRQAPGKEREFVAGI | TWTAGRTY | 59 |

29  30                                                    58

|  | FR3 | CDR3 | FR4 | |
|---|---|---|---|---|
|  | YADVASGRFTISRDNAKNTVYLQMNSLQPEDTAVYYCAA | KYG GSARSR T YD- | YWGQGTQVTVS | 123 |
|  | YADSVKGRFTISRDNAKNTVYLQMNSLKPEDTAVYYCAA | - G GYSRRW SA-YN- | YWGQGTQVTVS | 122 |
|  | YADSVKGRFTISKDNVKNMAYLQMNSLKPEDTGVYYCAA | TSS GSTRQL STGRSN | YWGQGTQVTVS | 126 |
|  | YADSVKGRFTISRDNAKRTTYLQMNSLKPEDTGVYYCAA | TSS GSTRLL STGRDN | YWGQGTLVTVS | 124 |

104  107  110
105

FIG 3

FIG 4A

FIG 4B

FIG 5

FIG 6

FIG 7

FIG 8

FIG 9

DAPI  VHH          DAPI  VHH

S2

S12

C25

C8

FIG 10

10 μm

FIG 11A

FIG 11B

FIG 12

FIG 13

FIG 14

FIG 15

FIG 16

FIG 17

FIG 18

FIG 19

FIG 20

FIG 21

FIG 22

FIG 23

Pourcentage des cellules avec des foyers 53BP1 (%)

FIG 24

Pourcentage des cellules avec des foyers BRCA1 (%)

FIG 25

Pourcentage des cellules avec du signal γH2AX (%)

FIG 26

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ANDERSON et al.** *Protein expression and purification,* 2010, vol. 72 (2), 194-204
- **ARBABI GHAHROUDI et al.** *FEBS Lett.,* 1997, vol. 414 (3), 521-6
- **BISCHOF et al.** *Proc. Natl. Acad. Sci. U. S. A.,* 2007, vol. 104, 3312-3317
- **CHIU et al.** *Molecular Pharmacology,* 2010, vol. 77 (4), 497-507
- **KIRSCH-VOLDERS.** *Mutation Res.,* 1997, vol. 392, 1-4
- **LEE et al.** *Nature Protocols,* 2007, vol. 2 (11), 3001-8
- **MAILAND et al.** *Cell,* 2007, vol. 131 (5), 887-900
- **PHELPS et al.** *Methods,* 1998, vol. 14, 367-379
- **SHECHTER et al.** *Nature Protocols,* 2007, vol. 2 (6), 1445-57
- **SIRVEN et al.** *Mol Ther.,* 2001, vol. 3 (4), 438-48